(19) Europäisches Patentamt
European Patent Office
Office européen des brevets

(11) **EP 3 741 379 A1**

(12) **EUROPEAN PATENT APPLICATION**
published in accordance with Art. 153(4) EPC

(43) Date of publication:
**25.11.2020 Bulletin 2020/48**

(51) Int Cl.:
*A61K 36/58* (2006.01)   *A61K 9/107* (2006.01)
*A61P 17/10* (2006.01)   *A61P 17/18* (2006.01)
*A61P 31/04* (2006.01)   *A61K 8/9789* (2017.01)
*A61K 8/06* (2006.01)   *A61Q 19/00* (2006.01)
*A61Q 19/02* (2006.01)   *A61Q 19/08* (2006.01)

(21) Application number: **19914695.2**

(22) Date of filing: **20.12.2019**

(86) International application number:
**PCT/KR2019/018216**

(87) International publication number:
**WO 2020/171364 (27.08.2020 Gazette 2020/35)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **20.02.2019 KR 20190019625**

(71) Applicant: HONEST CO.,LTD.
**Gyeongsangbuk-do 38578 (KR)**

(72) Inventors:
• **KWAK, Ki Sung
Daegu 42279 (KR)**
• **KIM, Hyun Jeong
Daegu 42166 (KR)**
• **LEE, Ji Hye
Daegu 42247 (KR)**
• **KANG, Eun Bin
Gyeongsan-si
Gyeongsangbuk-do 38647 (KR)**
• **GU, Su Ran
Gyeongsan-si
Gyeongsangbuk-do 38623 (KR)**

(74) Representative: HGF Europe LLP
**Neumarkter Str. 18
81673 München (DE)**

(54) **COSMETIC COMPOSITION COMPRISING AZADIRACHTA INDICA LEAF EXTRACT**

(57) The present invention relates to a cosmetic composition containing an *Azadirachta indica* leaf extract, and particularly to a cosmetic composition containing an *Azadirachta indica* leaf extract as an active ingredient and having antibacterial efficacy, anti-inflammatory efficacy and anti-pollution efficacy, in which the anti-pollution efficacy prevents skin adsorption of air pollutants, fine dust or heavy metal particles and protects skin cells, a cosmetic product composition including the cosmetic composition, and a Pickering emulsion composition containing an *Azadirachta indica* leaf extract.

FIG. 9a

**Description**

**Technical Field**

The present invention relates to a cosmetic composition containing an *Azadirachta indica* leaf extract, a cosmetic product composition including the same, and a Pickering emulsion composition containing an *Azadirachta indica* leaf extract.

**Background Art**

Fine dust, which is very fine particulate material having a diameter of 10 $\mu$m or less, has a great influence on the skin. When fine dust sticks to the skin and combines with skin oil, waste accumulates on the skin and penetrates into the pores, and moreover, heavy metals such as arsenic, cadmium, lead and the like may cause skin troubles such as acne and pimples when penetrating in this way. In particular, fine dust is more harmful to patients with skin troubles such as acne and the like. Acne disease is rapidly increasing in recent years, and the range of ages affected by acne has expanded to include not only teens but also people in their 20s. Here, environmental pollution due to the fine dust mentioned above is known to be the biggest cause of acne, along with westernization of eating habits and an increase in stress of modern people.

Currently, anti-acne cosmetics are mainly used to reduce skin troubles due to fine dust, etc., and most thereof are products containing a disinfectant as a main component and have not been widely adopted due to concerns about side effects thereof. Also, in order to solve skin problems caused by fine dust, there are methods of frequently washing face after returning home or of applying skin-protective cosmetics to prevent direct contact with fine dust. Most of conventionally developed cosmetics related to fine dust are cleansing products in which fine foam is used to remove fine dust or various pollutants including makeup residue. However, some fine dust in contact with the skin may be removed through facial cleansing, but it is very difficult to remove fine dust that has penetrated into the skin through routes such as pores, etc. Therefore, it is more important to protect the skin by preventing fine dust from contacting or penetrating into the skin.

Meanwhile, various factors act on human aging. In particular, reactive oxygen species, which are a factor that promotes aging as well as various diseases in the human body, are generated and react with DNA, cell membranes, and major constituents of the skin such as proteins, polysaccharides and lipids, causing a cascade of oxidative reactions, undesirably resulting in damaged cells and skin aging. Thorough research into functional foods and cosmetics for removing such reactive oxygen species is ongoing.

Also, ultraviolet (UV) light is known to promote the production of melanin, and excessive synthesis and accumulation of melanin may lead to visually unfavorable diseases such as liver spots and freckles. Melanosomes contain the enzymes necessary to synthesize normal melanin, and typical examples thereof may include tyrosinase-related protein-1 (TRP-1), tyrosinase, tyrosinase-related protein-2 (TRP-2) and the like.

Oxidative stress caused by excess reactive oxygen species (ROS) damages cells externally, and aging and pathological damage may occur due to inflammation caused by oxidative stress internally. An inflammatory response is a normal defense mechanism that occurs *in vivo* in order to respond to external stimuli, and when an inflammatory response occurs, inducible nitric oxide synthase (iNOS), tumor necrosis factor-$\alpha$ (TNF-$\alpha$), nitric oxide (NO), interleukin-1$\beta$ (IL-1$\beta$) and cyclooxygenase-2 (COX-2) are secreted. Research into aging and inflammatory diseases caused by oxidative stress is continually underway.

As the skin ages, the skin becomes thinner, more wrinkled, and less elastic. This is a phenomenon that is caused by a decrease in the secretion of hormones that regulate the body metabolism, and a reduction in biosynthesis of proteins that constitute the skin due to the lowered cellular activity of the skin. Moreover, photoaging changes such as deep wrinkles and thick skin are caused by free radicals and harmful reactive oxygen species, content of which is increased upon exposure to UV light. As such aging progresses, the ability to produce structural proteins such as collagen, elastin and hyaluronic acid, which make up the skin, decreases, and biosynthesis of type I collagenase (matrix metalloproteinase; MMP) increases, thus causing further degradation of skin structural proteins. Moreover, this accumulated oxidative stress further accelerates aging in the skin. Among protein enzymes that increase in content upon exposure to UV light, matrix metalloproteinase (MMP), which degrades a matrix, plays an important role in the process of photoaging of the skin. The extracellular matrix in the dermis of the skin functions to maintain the structure and elasticity of the skin, 80% or more of which is composed of type I procollagen. In skin exposed to UV light, the expression of MMP increases and the increased MMP degrades collagen constituting the skin, thus causing a lack of matrix protein, resulting in aged skin.

Meanwhile, the Neem tree, also called 'Neem' or the 'Nim tree', is an evergreen tree of the family *Meliaceae,* and has the scientific name *Azadirachta indica.* It is found in tropical and subtropical regions in India, Nepal, Pakistan, Bangladesh, Sri Lanka, and the Maldives, has the feature of rapidly growing to 15-20 m in height, and is used for pesticides, dental hygiene, desertification prevention and traditional medicine in the Indian continent.

## Disclosure

### Technical Problem

The present inventors have studied extracts effective for aging due to internal and external factors such as reactive oxygen species, UV light, fine dust, etc., and have ascertained that an *Azadirachta indica* leaf extract has efficacy on skin aging or wrinkles caused by reactive oxygen species, melanin production promotion caused by UV light, skin aging and skin diseases caused by fine dust, and the like, thus culminating in the present invention.

Accordingly, an objective of the present invention is to provide a cosmetic composition containing an *Azadirachta indica* leaf extract as an active ingredient and a cosmetic product composition including the same.

Another objective of the present invention is to provide a Pickering emulsion that may be utilized in various industries such as foods, cosmetic products, pharmaceutical products, etc., using the pharmacologically active effect of the *Azadirachta indica* leaf extract.

The objectives of the present invention are not limited to the foregoing, and other objectives not mentioned herein will be able to be clearly understood by those skilled in the art from the following description.

### Technical Solution

In order to accomplish the above objectives, the present invention provides a cosmetic composition containing an *Azadirachta indica* leaf extract as an active ingredient and having antibacterial efficacy, anti-inflammatory efficacy and anti-pollution efficacy, in which the anti-pollution efficacy prevents skin adsorption of air pollutants, fine dust or heavy metal particles and protects skin cells.

In the present invention, the anti-pollution efficacy serves to prevent the degradation of hyaluronic acid to thus prevent skin adsorption, and the antibacterial efficacy serves to inhibit the growth of *Staphylococcus aureus* and *Propionibacterium acnes.*

Also, in the present invention, the cosmetic composition may additionally have antioxidant efficacy, whitening efficacy, and wrinkle reduction efficacy.

Also, in the present invention, the *Azadirachta indica* leaf extract may be an *Azadirachta indica* leaf hot-water extract, an *Azadirachta indica* leaf ethanol extract or an *Azadirachta indica* leaf acetone extract.

In addition, the present invention provides a cosmetic product composition including the cosmetic composition containing the *Azadirachta indica* leaf extract as the active ingredient. Here, the cosmetic product composition is preferably in the form of an essence, ampoule, soap, tonic, body essence, emulsion, lotion, cream (oil-in-water, water-in-oil or multi-phase), solution, suspension (anhydrous or hydrous), anhydrous product (oil or glycol), gel, or powder.

In addition, the present invention provides a Pickering emulsion composition including an *Azadirachta indica* leaf extract as an active ingredient, further including a powder phase, a water phase and an additive, and having antibacterial efficacy, anti-inflammatory efficacy and anti-pollution efficacy, in which the anti-pollution efficacy prevents skin adsorption of air pollutants, fine dust or heavy metal particles and protects skin cells.

In the present invention, the Pickering emulsion composition may be prepared by subjecting a composition including the *Azadirachta indica* leaf extract extracted using hot water, ethanol or acetone, the powder phase, the water phase and the additive to pulverization at 10,000 rpm to 20,000 rpm for 30 sec to 5 min and then mixing at 300 to 800 rpm for 1 to 5 min.

Also, in the present invention, the anti-pollution efficacy serves to prevent the degradation of hyaluronic acid to thus prevent skin adsorption, and the antibacterial efficacy serves to inhibit the growth of *Staphylococcus aureus* and *Propionibacterium acnes.*

Also, in the present invention, the Pickering emulsion composition may additionally have antioxidant efficacy, whitening efficacy, and wrinkle reduction efficacy.

### Advantageous Effects

According to the present invention, a cosmetic composition containing an *Azadirachta indica* leaf extract as an active ingredient is effective at preventing skin adsorption of air pollutants, fine dust or heavy metal particles and protecting skin cells, thereby exhibiting anti-pollution efficacy. Thus, it has a significant effect on skin diseases or skin inflammation due to adsorption of pollutants, fine dust, heavy metals, etc., has antibacterial effects, improves the health of skin cells, and aids in maintaining skin moisturization and barrier functions. Therefore, the cosmetic composition of the present invention containing the *Azadirachta indica* leaf extract as an active ingredient can exhibit antibacterial efficacy, anti-inflammatory efficacy, and anti-pollution efficacy, and additionally, antioxidant efficacy, whitening efficacy, and wrinkle reduction efficacy.

Moreover, in the present invention, the antioxidant effect, antibacterial effect, anti-inflammatory effect (acne reduction), whitening effect (skin pigmentation reduction), wrinkle reduction effect, etc. of the *Azadirachta indica* leaf extract are verified for various types of solvent used therefor, thereby increasing the variety of possible end uses.

In addition, the present invention provides a composition containing an active ingredient extracted from *Azadirachta indica* leaves, and can be utilized for foods, cosmetic products, pharmaceutical products, etc. In particular, when used for cosmetic products, it is possible to provide a Pickering emulsion composition in which the active ingredient and other additives are contained in a more stable form. Accordingly, it is possible to improve the sensation of use while promoting percutaneous absorption of the *Azadirachta indica* leaf extract as the active ingredient.

**Brief Description of Drawings**

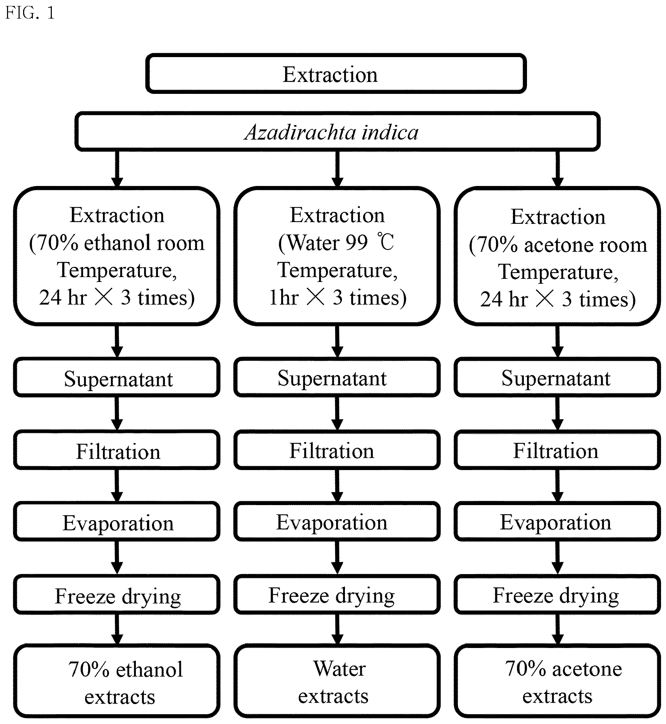

FIG. 1 shows a process of extracting *Azadirachta indica* according to an embodiment of the present invention, in which AIW represents the *Azadirachta indica* leaf hot-water extract, AIE represents the *Azadirachta indica* leaf ethanol extract, and AIA represents the *Azadirachta indica* leaf acetone extract;

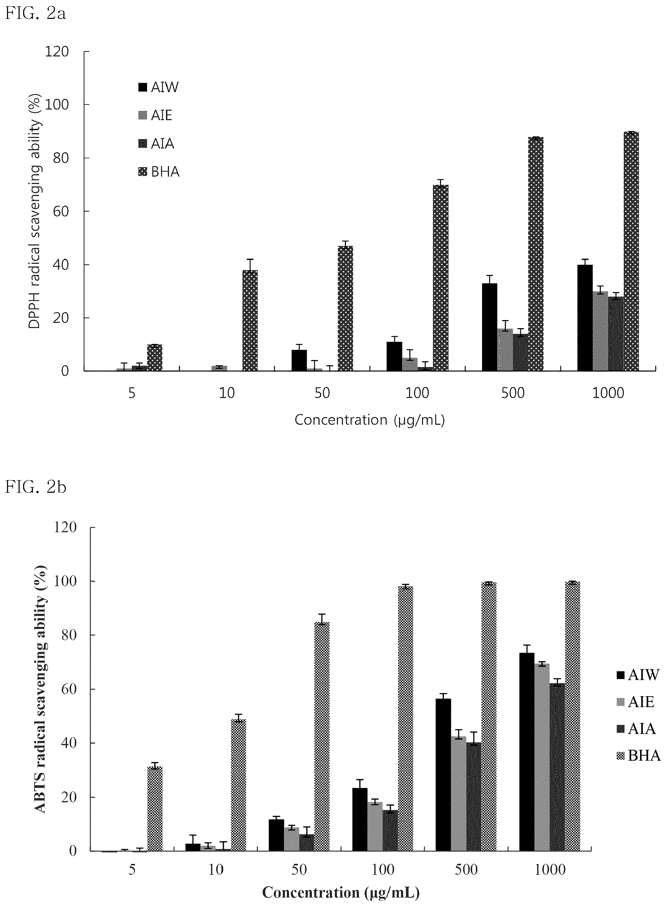

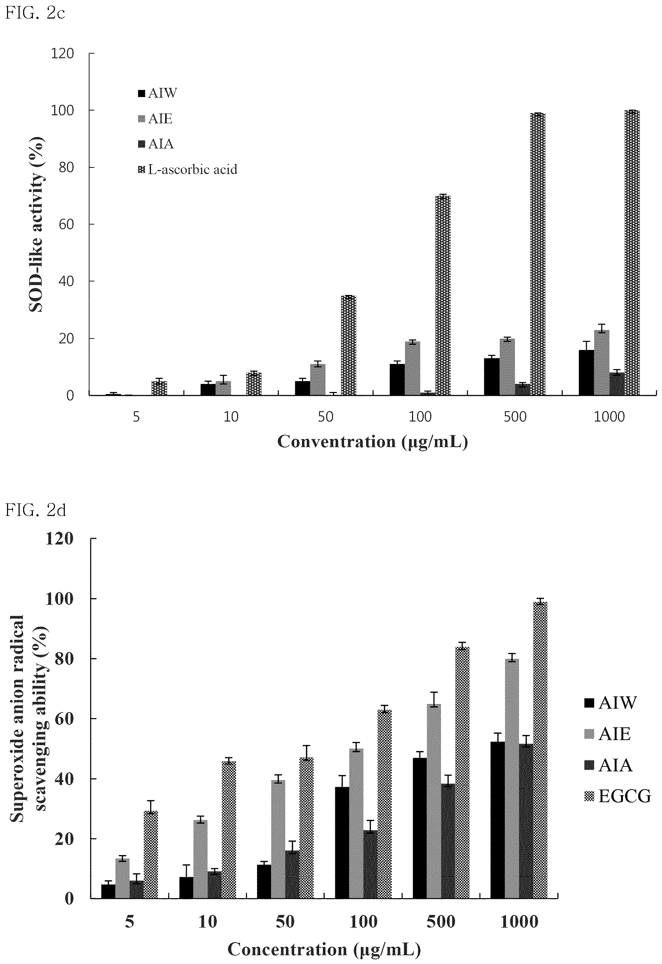

FIG. 2 shows the results of measurement of antioxidant effect of the *Azadirachta indica* leaf extract in Experimental Example 1 of the present invention, FIG. 2a showing the results of measurement of DPPH radical scavenging ability, FIG. 2b showing the results of measurement of ABTS cation radical scavenging ability, FIG. 2c showing the results of measurement of SOD-like activity, and FIG. 2d showing the results of measurement of superoxide anion radical scavenging ability;

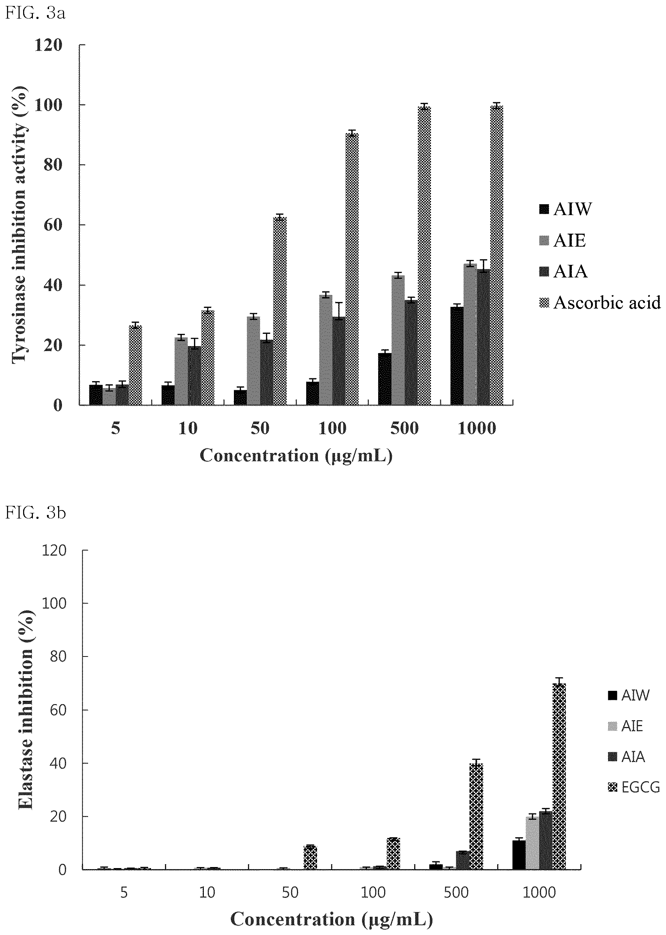

FIG. 3a shows the results of measurement of the effect of the *Azadirachta indica* leaf extract on tyrosinase inhibitory activity in Experimental Example 3 of the present invention, and FIG. 3b shows the results of measurement of the effect of the *Azadirachta indica* leaf extract on elastase inhibitory activity in Experimental Example 4 of the present invention;

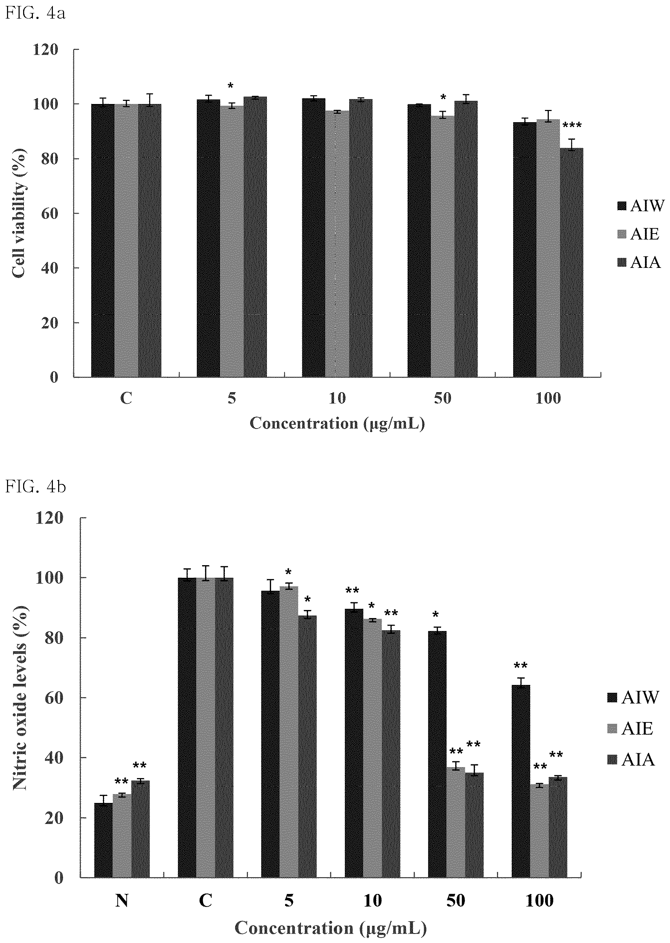

FIG. 4 shows the results of measurement of the effect of the *Azadirachta indica* leaf extract on cytotoxicity and the anti-inflammatory effect thereof in Experimental Example 5 of the present invention, FIG. 4a showing the results of measurement of macrophage cell viability and FIG. 4b showing the results of measurement of NO inhibitory activity;

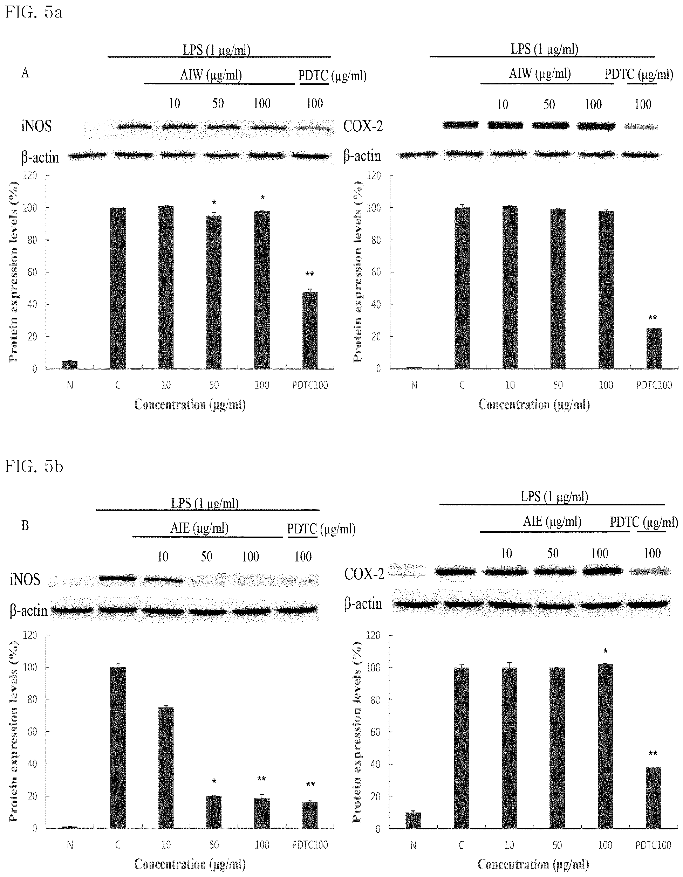

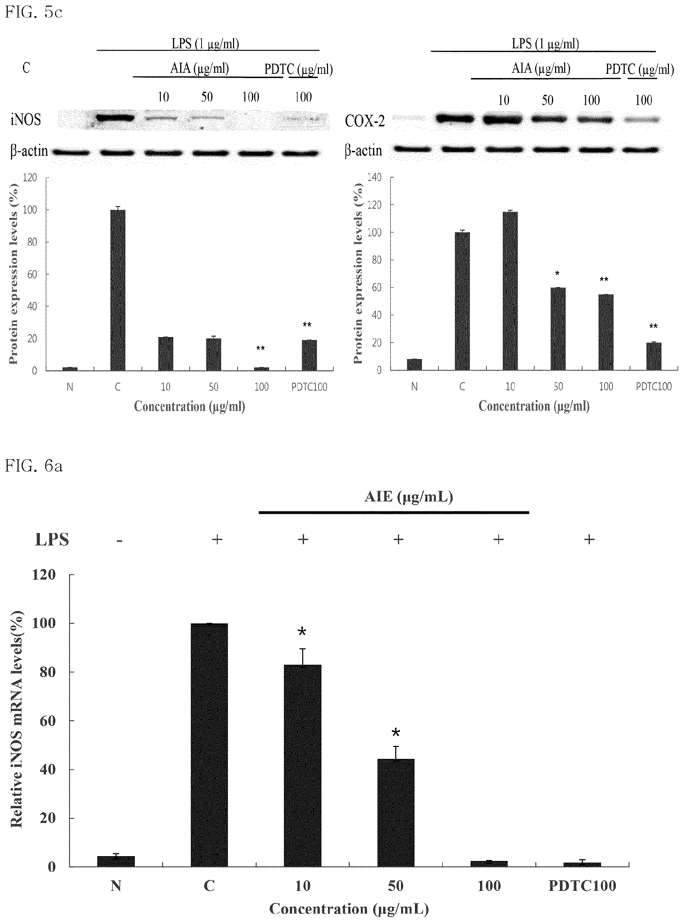

FIG. 5 shows the results of measurement of anti-inflammatory effect of the *Azadirachta indica* leaf extract through Western blot in Experimental Example 5 of the present invention, FIGS. 5a, 5b and 5c respectively showing the results of measurement of the effects of AIW, AIE and AIA on inhibiting the expression of iNOS and COX-2;

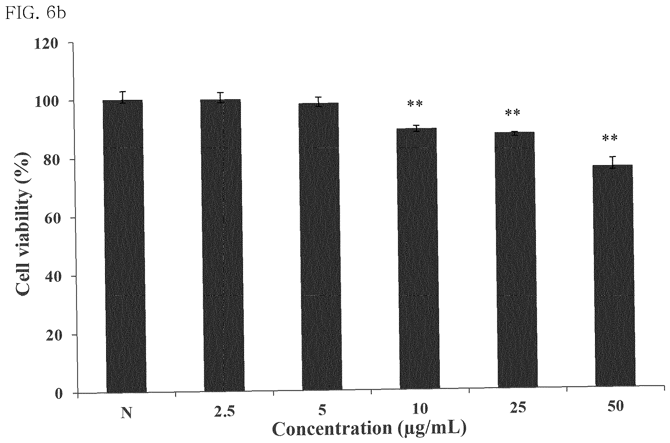

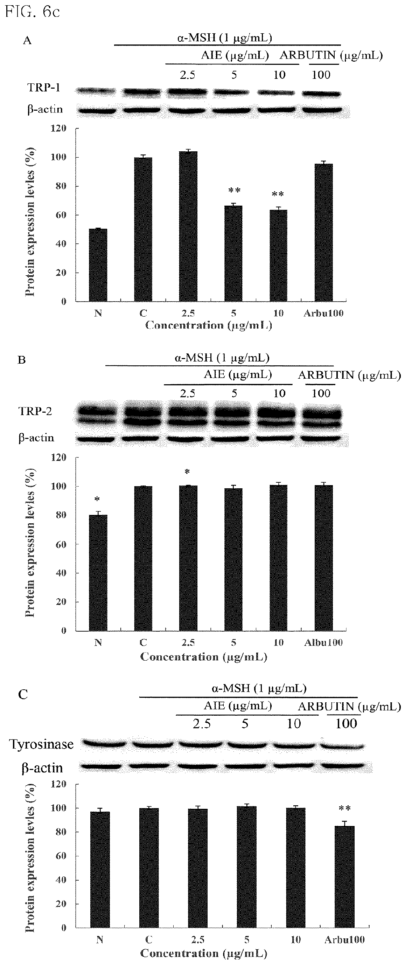

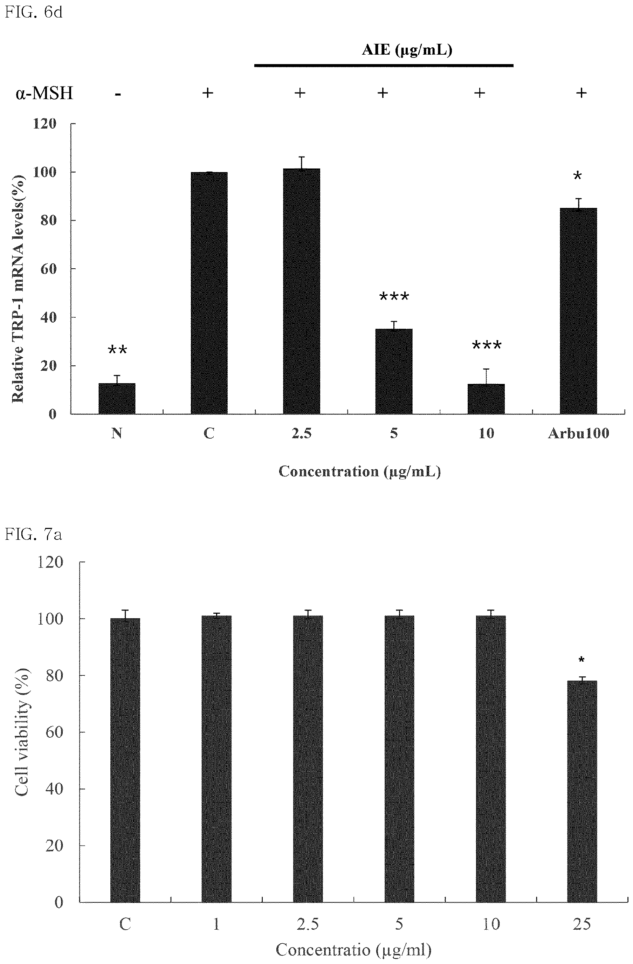

FIG. 6 shows the results of measurement by AIE in Experimental Example 5 of the present invention, FIG. 6a showing the results of measurement of inhibition of iNOS mRNA expression, FIG. 6b showing the results of measurement of melanoma cell (B16F10) cytotoxicity, FIG. 6c showing the results of measurement of inhibition of TRP-1 (A), TRP-2 (B) and tyrosinase (C) expression, and FIG. 6d showing the results of measurement of inhibition of TRP-1 mRNA expression;

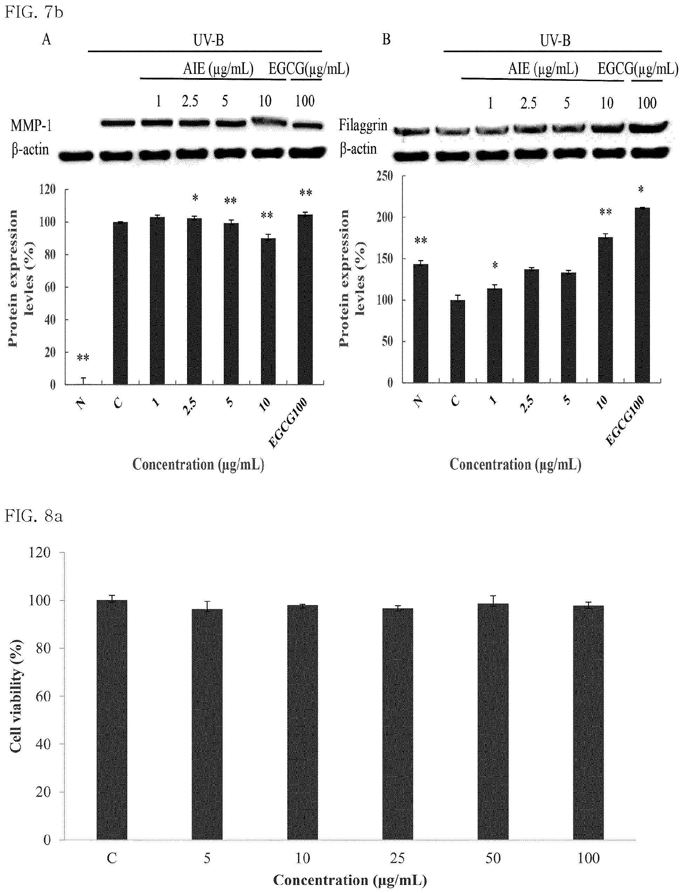

FIG. 7 shows the results of measurement of the wrinkle reduction effect of AIE in Experimental Example 7 of the present invention, FIG. 7a showing the results of measurement of cell viability of human keratinocytes and FIG. 7b showing the results of measurement of MMP-1 and filaggrin protein expression;

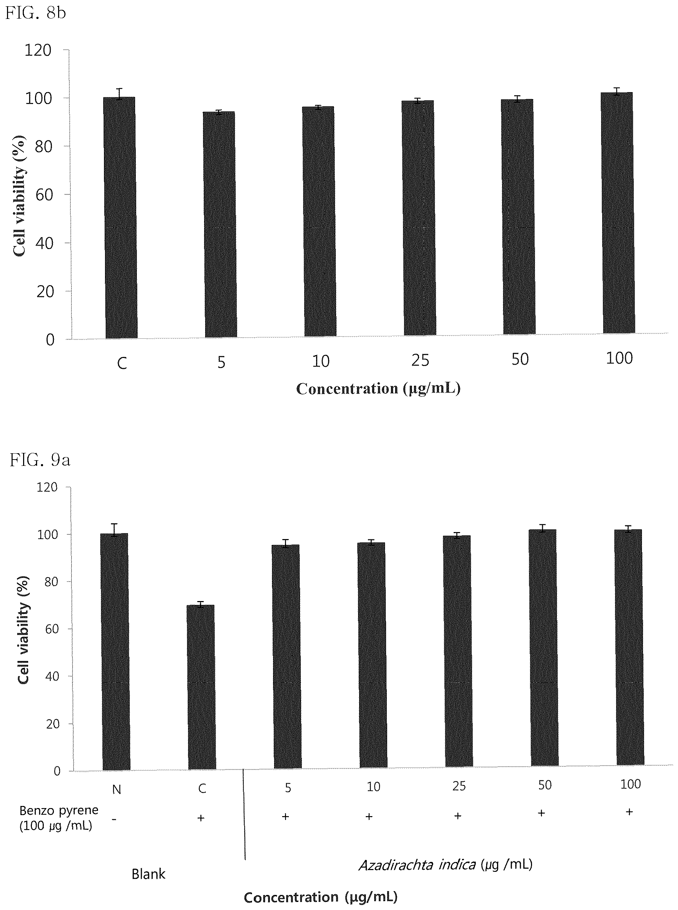

FIG. 8 shows the results of measurement of cytotoxicity in Experimental Example 8 of the present invention, FIG. 8a showing cell viability of keratinocytes (HaCaT cells), and FIG. 8b showing cell viability of fibroblasts (CCD-986sk cells);

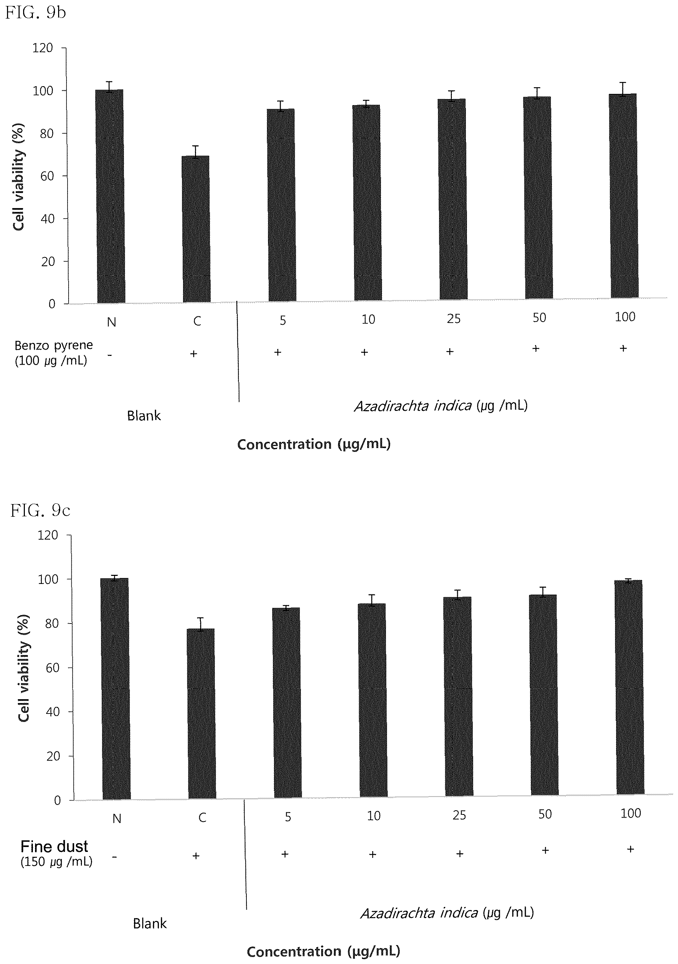

FIG. 9 shows the results of an anti-pollution experiment in Experimental Example 8 of the present invention, FIG. 9a showing the results of measurement of cell viability of keratinocytes (HaCaT cells) by the *Azadirachta indica* leaf extract when treated with benzo[$\alpha$]pyrene (BP), FIG. 9b showing the results of measurement of cell viability of fibroblasts (CCD-986sk cells) by the *Azadirachta indica* leaf extract when treated with benzo[$\alpha$]pyrene (BP), and FIG. 9c showing the results of measurement of cell viability of fibroblasts (CCD-986sk cells) by the *Azadirachta indica* leaf extract when treated with fine dust;

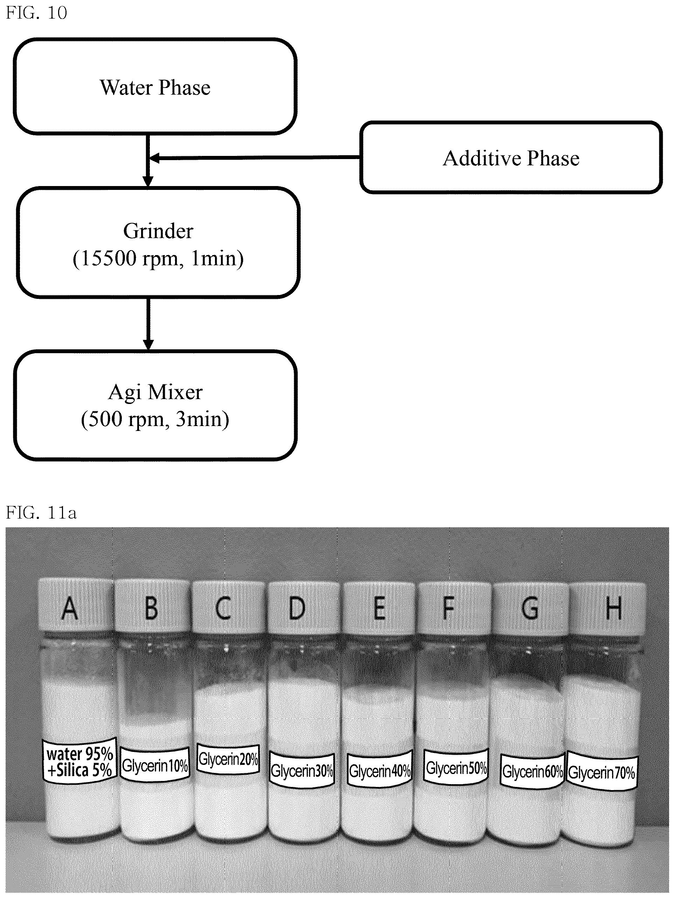

FIG. 10 shows a process of preparing a Pickering emulsion in Experimental Example 9 of the present invention;

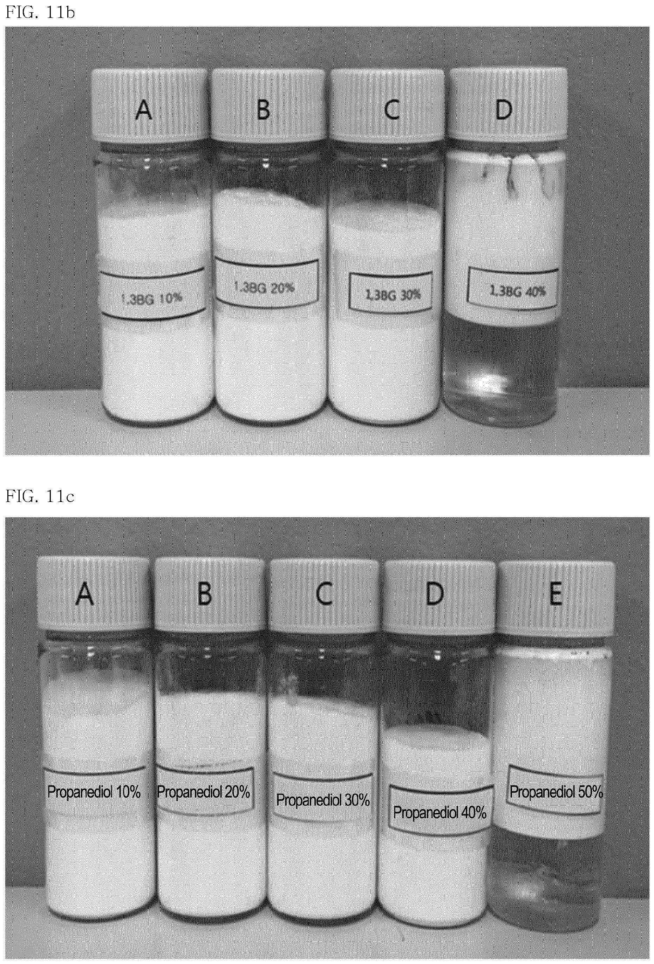

FIG. 11 shows the formulation change depending on the amount of moisturizer of the Pickering emulsion in Experimental Example 9 of the present invention, FIG. 11a showing the formulation change depending on the amount of glycerin, FIG. 11b showing the formulation change depending on the amount of 1,3-BG, and FIG. 11c showing the formulation change depending on the amount of propanediol;

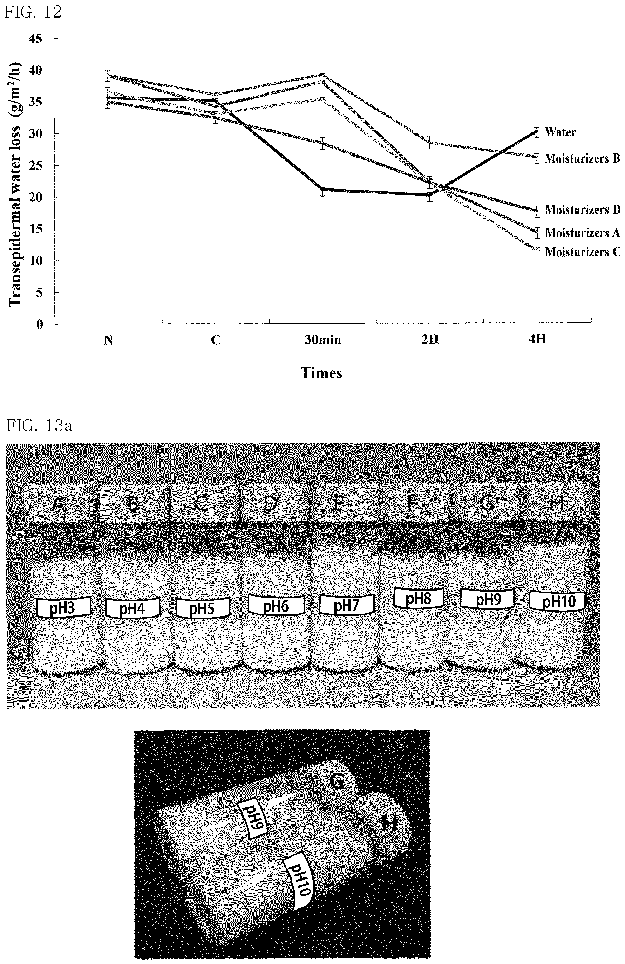

FIG. 12 shows the results of measurement of transepidermal water loss (TEWL) depending on the amount of composite moisturizer of the Pickering emulsion in Experimental Example 9 of the present invention; and

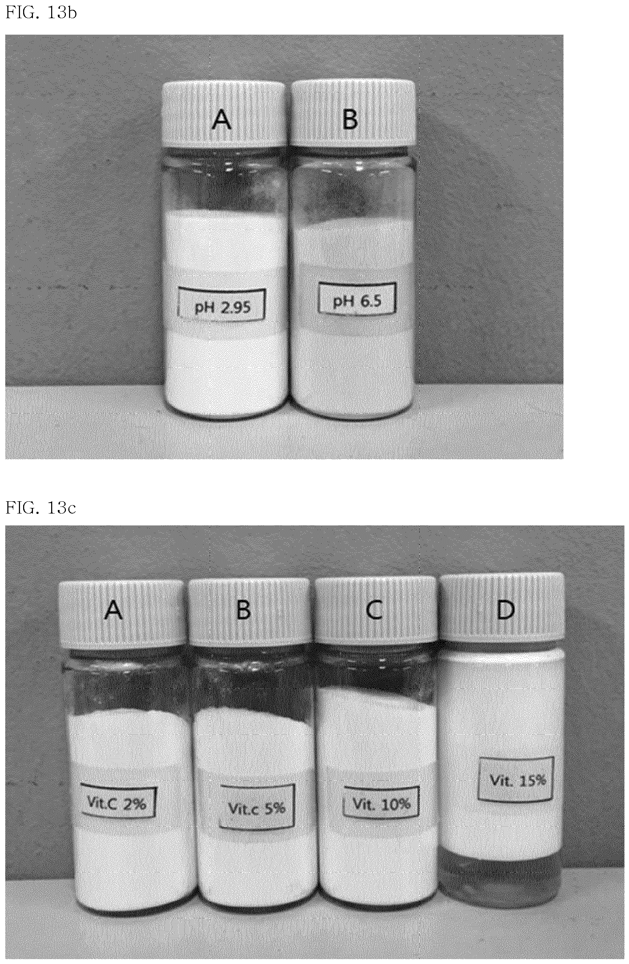

FIG. 13 shows the formulation change depending on the pH in Experimental Example 9 of the present invention, FIG. 13a showing the formulation change depending on the pH of the Pickering emulsion, FIG. 13b showing the formulation change depending on the pH of L-ascorbic acid, and FIG. 13c showing the formulation change depending on the amount of L-ascorbic acid.

**Best Mode**

In the following description of the present invention, the terms used herein are merely intended to describe specific embodiments, and are not to be construed as limiting the scope of the present invention, which is defined by the appended claims. Unless otherwise defined, all technical or scientific terms used herein have the same meanings as those typically understood by persons having ordinary knowledge in the art to which the present invention belongs.

Unless otherwise stated, the terms "comprise", "comprises" and "comprising" are used to designate the presence of an object or step or groups of objects or steps described in the specification and claims, and should be understood as not excluding the presence or additional possibility of inclusion of any other objects, steps or groups of objects or steps.

Unless otherwise noted, various embodiments of the present invention may be combined with other embodiments. In particular, any feature that is said to be preferable or favorable may be combined with any other features said to be preferable or favorable.

According to an embodiment of the present invention, the composition contains an *Azadirachta indica* leaf extract, particularly an active ingredient extracted from the plant, which may be used for cosmetic products that protect the skin from fine dust, heavy metals, etc., functional cosmetic products for acne suppression and skin pigmentation reduction, foods, pharmaceutical products, etc. As used herein, the term "active ingredient" refers to a component that may exhibit the desired activity when used alone, or may exhibit activity together with a carrier which is itself inactive.

An aspect of the present invention pertains to a cosmetic composition containing an *Azadirachta indica* leaf extract as an active ingredient.

In the present invention, the *Azadirachta indica* leaf extract may be obtained using a solvent extraction process, and the solvent used for the solvent extraction process may be selected from the group consisting of, for example, water, a C1-C4 anhydrous or hydrous lower alcohol (e.g. methanol, ethanol, propanol, or butanol), propylene glycol, 1,3-butylene glycol, glycerin, acetone, diethyl ether, ethyl acetate, butyl acetate, dichloromethane, chloroform, hexane and mixtures thereof.

Preferably, the *Azadirachta indica* active ingredient may be extracted using hot water, ethanol or acetone. Here, the *Azadirachta indica* leaf extract is obtained in a manner in which an *Azadirachta indica* leaf is immersed in the solvent, such as hot water, ethanol or acetone, and is then extracted. The extraction process may be performed differently depending on the solvent. Specifically, when the *Azadirachta indica* leaf is extracted using hot water, a dried *Azadirachta indica* leaf is immersed in hot water in an amount corresponding to 8 to 12 times the weight of the dried leaf and then extracted at 90 to 110°C for 3 to 5 hr. Here, the above extraction step may be repeated at least two times, and preferably three times or more, thus obtaining an extract. Alternatively, when the *Azadirachta indica* leaf is extracted using ethanol or acetone, a dried *Azadirachta indica* leaf is immersed in a 60-80% ethanol or acetone solution serving as a solvent in an amount corresponding to 8 to 12 times the weight of the dried leaf, and is then extracted at room temperature for 20 to 30 hr. Here, the above extraction step may be repeated at least two times, and preferably three times or more, thus obtaining an extract.

Moreover, the *Azadirachta indica* leaf extract containing the extracted *Azadirachta indica* active ingredient exhibits an antibacterial effect, anti-inflammatory effect (acne reduction) and anti-pollution effect.

Here, with regard to the antibacterial effect, the *Azadirachta indica* leaf extract inhibits the growth of *Staphylococcus aureus* and *Propionibacterium acnes,* and thus has an antibacterial effect. More specifically, in an experiment for measuring inhibitory zones, the *Azadirachta indica* leaf ethanol or acetone extract exhibits a clear zone of $1.0\pm0$ cm or more from 2 mg/disc in *Staphylococcus aureus,* and all *Azadirachta indica* leaf extracts show clear zones of $1.1\pm0$ cm or more from 5 mg/disc in *Propionibacterium acnes.*

Also, with regard to the anti-inflammatory effect, the *Azadirachta indica* leaf extract exhibits RAW 264.7 macrophage cell viability of 90% or more, NO inhibitory activity, and iNOS and COX-2 protein expression inhibitory activity, and thus has an anti-inflammatory effect.

In particular, the *Azadirachta indica* leaf ethanol or acetone extract exhibits superior NO inhibitory activity of 65% or more at a concentration of 100 $\mu$g/mL, and the NO inhibitory activity of the *Azadirachta indica* leaf ethanol extract is the greatest. Moreover, the *Azadirachta indica* leaf ethanol extract has iNOS protein expression inhibitory activity, and the *Azadirachta indica* leaf acetone extract has iNOS and COX-2 protein expression inhibitory activity. More specifically, the *Azadirachta indica* leaf ethanol extract exhibits the highest iNOS mRNA expression inhibitory activity of 97% or more at a concentration of 100 $\mu$g/mL.

Also, with regard to the anti-pollution effect, the *Azadirachta indica* leaf extract has anti-pollution efficacy in which the health of human skin cells such as HaCaT cells and CCD-986sk cells, stimulated with benzo[$\alpha$]pyrene (BP), is improved by the *Azadirachta indica* extract. Here, benzopyrene, which is a kind of polycyclic aromatic hydrocarbon generated in the process of incomplete combustion of fossil fuel or the like, is a pollutant contained in the air as well as soot. In particular, an improvement effect of HaCaT cells is 92% or more when using the *Azadirachta indica* extract at 25 μg/mL, and an improvement effect of CCD-986sk cells is 81% or more when using the *Azadirachta indica* extract at 25 μg/mL, compared to the cells treated with benzo[α]pyrene alone.

Also, the *Azadirachta indica* leaf extract has anti-pollution efficacy of protecting skin cells in which the health of CCD-986sk cells stimulated with fine dust is improved by the *Azadirachta indica* extract. Specifically, an improvement effect of CCD-986sk cells is 85% or more when using the *Azadirachta indica* extract at 100 μg/mL, compared to the cells treated with fine dust alone.

Also, the *Azadirachta indica* leaf extract has an effect of protecting hyaluronic acid, which plays a role in forming a skin barrier. According to an embodiment of the present invention, based on the results of measurement of the viscosity of hyaluronic acid after reaction of 10 ppm iron, 1% *Azadirachta indica* extract and 0.2% EDTA-2Na with hyaluronic acid, which forms a skin barrier and is involved in skin moisturization, it is confirmed that the *Azadirachta indica* extract has a better effect of binding to iron than EDTA-2Na, resulting in higher viscosity. This shows that the *Azadirachta indica* leaf extract is capable of preventing the degradation of hyaluronic acid and also that the effect thereof is high compared to EDTA-2Na, which adsorbs heavy metals, etc.

Accordingly, the *Azadirachta indica* leaf extract is contained in a cosmetic product composition, thus preventing skin adsorption of air pollutants, fine dust or heavy metal particles and protecting skin cells, thereby manifesting anti-pollution efficacy. Therefore, it has a significant effect on skin diseases or skin inflammation due to adsorption of pollutants, fine dust, heavy metals, etc., has antibacterial effects, improves the health of skin cells, and aids in maintaining skin moisturization and barrier functions.

Moreover, the *Azadirachta indica* leaf extract containing the extracted *Azadirachta indica* active ingredient exhibits antioxidant, whitening, and wrinkle reduction effects, in addition to the aforementioned antibacterial effect, anti-inflammatory effect (acne reduction), and anti-pollution effect.

With regard to the antioxidant effect, the *Azadirachta indica* leaf extract has high polyphenol content of 50 mg/g or more, and exhibits DPPH radical scavenging ability, ABTS$^+$ radical scavenging ability, SOD-like activity, and superoxide anion radical scavenging ability, and thus has an antioxidant effect.

In particular, the *Azadirachta indica* leaf hot-water extract exhibits DPPH radical scavenging ability of 40% or more at a concentration of 1,000 μg/mL, and the ABTS$^+$ radical scavenging ability thereof is 70% or more, which is regarded as the greatest. The *Azadirachta indica* leaf ethanol extract exhibits SOD-like activity of 20% or more at a concentration of 1,000 μg/mL, and the superoxide anion radical scavenging ability thereof is 95% or more, which is regarded as the greatest. The *Azadirachta indica* leaf acetone extract has the highest polyphenol content of 70 mg/g or more.

Also, with regard to the whitening effect, the *Azadirachta indica* leaf extract exhibits tyrosinase inhibitory activity, thereby showing a skin pigmentation reduction effect, that is, a whitening effect. In particular, the *Azadirachta indica* leaf ethanol or acetone extract has superior tyrosinase inhibitory activity of 45% or more at a concentration of 1,000 μg/mL, and the tyrosinase inhibitory activity of the *Azadirachta indica* leaf ethanol extract is the greatest.

Furthermore, the *Azadirachta indica* leaf ethanol extract, having the greatest tyrosinase inhibitory activity, exhibits B16F10 melanoma cell viability of 85% or more, and has high TRP-1 mRNA expression inhibitory activity of 85% or more at a concentration of 10 μg/mL.

Also, with regard to the wrinkle reduction effect, the *Azadirachta indica* leaf extract exhibits elastase inhibitory activity and thus has a wrinkle reduction effect. In particular, the *Azadirachta indica* leaf ethanol or acetone extract exhibits superior elastase inhibitory activity of 20% or more at a concentration of 1,000 μg/mL, and the elastase inhibitory activity of the *Azadirachta indica* leaf ethanol extract is the greatest.

Moreover, the *Azadirachta indica* leaf ethanol extract, having the greatest elastase inhibitory activity, exhibits human keratinocyte cell viability of 90% or more, and has the ability to inhibit MMP-1 expression at a concentration of 10 μg/mL.

A cosmetic product, including the cosmetic composition of the present invention containing the *Azadirachta indica* leaf extract as the active ingredient and having antibacterial efficacy, anti-inflammatory efficacy, anti-pollution efficacy, antioxidant efficacy, whitening efficacy, and wrinkle reduction efficacy, as described above, may be provided. The cosmetic product containing the *Azadirachta indica* leaf extract may be manufactured in various formulations for skin and body care cosmetics, such as an essence, ampoule, soap, tonic, body essence, emulsion, lotion, cream (oil-in-water, water-in-oil, multi-phase), solution, suspension (anhydrous and hydrous), anhydrous product (oil and glycol), gel, powder, etc.

Here, the cosmetic product composition may include an acceptable carrier for skin cosmetic formulations, in addition to the *Azadirachta indica* leaf extract. Examples of the carrier may include alcohols, oils, surfactants, fatty acids, silicone oils, preservatives, wetting agents, moisturizers, viscosity modifiers, emulsifiers, stabilizers, sunscreens, colorants, fragrances, diluents, and the like.

The specific compounds or compositions that may be used for the alcohols, oils, surfactants, fatty acids, silicone oils, preservatives, wetting agents, moisturizers, viscosity modifiers, emulsifiers, stabilizers, sunscreens, colorants, fragrances, diluents, etc., are already known in the art, and thus, the corresponding compounds or compositions may be appropriately selected by those skilled in the art.

When exemplifying several materials herein, examples of the alcohol include water-soluble polyhydric alcohols such as higher alcohol, propylene glycol, 1,3-butylene glycol, glycerin, sorbitol, polyethylene glycol, etc., examples of the oil include avocado oil, palm oil, beef tallow, jojoba oil, etc., and examples of the preservative include ethylparaben, butylparaben, etc. Examples of the moisturizer include hyaluronic acid, chondroitin sulfate, pyrrolidone carboxylate, etc., and examples of the diluent include ethanol, isopropanol, etc.

More specifically, when the formulation for a cosmetic product is a paste, cream or gel, the carrier may include animal fiber, plant fiber, wax, paraffin, starch, tragacanth, cellulose derivatives, polyethylene glycol, silicone, bentonite, silica, talc or zinc oxide.

Also, when the formulation is a powder or spray, the carrier may include lactose, talc, silica, aluminum hydroxide, calcium silicate or polyamide powder. In particular, in the case of a spray, a propellant such as chlorofluorohydrocarbon, propane/butane or dimethyl ether may be additionally included.

Also, when the formulation is a solution or emulsion, the carrier may include a solvent, a solvating agent or an emulsifier, for example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or sorbitan fatty acid ester.

Also, when the formulation is a suspension, the carrier may include a liquid diluent such as water, ethanol or propylene glycol, a suspension agent such as ethoxylated isostearyl alcohol, polyoxyethylene sorbitol ester or polyoxyethylene sorbitan ester, microcrystalline cellulose, aluminum metahydroxide, bentonite, agar or tragacanth.

Also, when the formulation is a surfactant-containing cleanser, the carrier may include aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamido betaine, aliphatic alcohol, fatty acid glyceride, fatty acid diethanolamide, vegetable oil, lanolin derivative or ethoxylated glycerol fatty acid ester.

In addition, a soap containing the *Azadirachta indica* leaf extract according to an embodiment of the present invention may be manufactured by adding the *Azadirachta indica* leaf extract to a soap base. Here, an additive, including a skin moisturizer, an emulsifier, a water softener, etc., may be added thereto.

Examples of the soap base may include plant oils and fats, such as coconut oil, palm oil, soybean oil, castor oil, olive oil, and palm kernel oil, or animal oils and fats, such as beef tallow, pork fat, sheep fat, fish oil, and the like, and examples of the skin moisturizer may include glycerin, erythritol, polyethylene glycol, propylene glycol, butylene glycol, pentylene glycol, hexyl glycol, isopropyl myristate, silicone derivatives, aloe vera, sorbitol, and the like, examples of the emulsifier may include natural oil, wax, fatty alcohol, hydrocarbons, natural plant extract, and the like, and examples of the water softener may include tetrasodium EDTA, etc.

The soap composition of the present invention may further include an additive, such as an antibacterial agent, a dispersant, an antifoaming agent, a solvent, a descaling agent, a corrosion inhibitor, a fragrance, a colorant, a metal ion sequestering agent, an antioxidant, a preservative, etc.

Another aspect of the present invention pertains to a Pickering emulsion composition, which contains the *Azadirachta indica* leaf extract and has antibacterial efficacy, anti-inflammatory efficacy, anti-pollution efficacy, antioxidant efficacy, whitening efficacy and wrinkle reduction efficacy. The Pickering emulsion composition is an emulsion stabilized by solid particles, includes the *Azadirachta indica* leaf extract as the active ingredient, and further includes a powder phase, a water phase, and an additive, thereby promoting percutaneous absorption of the *Azadirachta indica* leaf extract as the active ingredient and improving the sensation of use thereof.

Here, the *Azadirachta indica* leaf extract may be the *Azadirachta indica* leaf hot-water extract, the *Azadirachta indica* leaf ethanol extract, or the *Azadirachta indica* leaf acetone extract, as described above. Among these, the extract having the greatest efficacy may be selectively used depending on the end use of the Pickering emulsion composition.

The *Azadirachta indica* leaf extract is included in an amount of 0.5 to 5 wt%, and preferably 0.5 to 3 wt%, based on the total weight of the Pickering emulsion composition.

The powder phase includes at least one selected from the group consisting of calcium carbonate, silica, clay, laponite, graphite, latex, magnetic particles and carbon nanotubes (CNTs), and preferably includes silica.

The powder phase is included in an amount of 1 to 10 wt%, and preferably 3 to 8 wt%, based on the total weight of the Pickering emulsion composition.

The water phase includes water and a moisturizer, and the moisturizer includes at least one selected from the group consisting of glycerin, 1,3-butylene glycol, sorbitol, ethylene glycol and propanediol. Preferably useful is a mixture of glycerin, 1,3-butylene glycol and propanediol.

The water phase is included in an amount of 75 to 95 wt%, and preferably 80 to 85 wt%, based on the total weight of the Pickering emulsion composition.

The water may be included in an amount of 50 to 80 wt%, and preferably 60 to 70 wt%, based on the total weight of the water phase. The moisturizer may be included in an amount of 20 to 50 wt%, and preferably 30 to 40 wt%, based on the total weight of the water phase.

More specifically, when glycerin is used as the moisturizer, it is included in an amount of 10 to 70 wt% based on the total weight of the water phase, and when 1,3-butylene glycol is used as the moisturizer, it is included in an amount of 10 to 30 wt% based on the total weight of the water phase. When propanediol is used as the moisturizer, it is included in an amount of 10 to 40 wt% based on the total weight of the water phase. Most preferably, a mixture of glycerin, 1,3-butylene glycol and propanediol is used as the moisturizer. Here, the above three components are mixed at a ratio of 1:0.8-1.2:0.8-1.2, and the resulting mixture may be included in an amount of 30 to 40 wt% based on the total weight of the water phase.

The additive includes at least one of an antioxidant, a preservative and a pH adjuster. Examples of the antioxidant include L-ascorbic acid, $\alpha$-tocopherol, polyphenol, carnosic acid, lipoic acid, 2-aminoethanesulfonic acid, aromatic carboxylic acid and combinations thereof, and examples of the preservative include 1,2-hexanediol, glyceryl caprylate, methylparaben, salicylic acid, isopropylparaben, phenoxyethanol and combinations thereof. Examples of the pH adjuster include citric acid and L-arginine.

The additive is included in an amount of 2.2 to 12 wt% based on the total weight of the Pickering emulsion composition. More specifically, the antioxidant is included in an amount of 2 to 10 wt% based on the total weight of the Pickering emulsion composition. The preservative is included in an amount of 0.1 to 1.0 wt%, and preferably 0.1 to 0.5 wt%, based on the total weight of the Pickering emulsion composition.

The pH adjuster is used such that the pH of the Pickering emulsion composition is adjusted to the range of 2 to 4, and is included in an amount of 0.1 to 1.0 wt%, and preferably 0.1 to 0.5 wt%, based on the total weight of the Pickering emulsion composition.

The Pickering emulsion composition is obtained by mixing the above components in the above amounts, followed by pulverization at 10,000 rpm to 20,000 rpm for 30 sec to 5 min and then mixing at 300 to 800 rpm for 1 to 5 min.

When the Pickering emulsion composition containing the *Azadirachta indica* leaf extract is used for a cosmetic product, it may be manufactured in various formulations for skin and body care products, such as essences, ampoules, soaps, tonics, body essences, emulsions, lotions, creams (oil-in-water, water-in-oil, multi-phase), solutions, suspensions (anhydrous and hydrous), anhydrous products (oil and glycol), gels, powders, and the like. The Pickering emulsion composition containing the *Azadirachta indica* leaf extract is included in an amount of 0.05 to 100 wt% based on the total weight of the cosmetic product. Here, an ampoule formulation may be composed of 100 wt% of the Pickering emulsion composition containing the *Azadirachta indica* leaf extract.

Also, the cosmetic product formulation containing the Pickering emulsion composition may further include, as an acceptable carrier, an alcohol, oil, surfactant, fatty acid, silicone oil, preservative, wetting agent, moisturizer, viscosity modifier, emulsifier, stabilizer, sunscreen, colorant, fragrance, diluent, etc., as described above.

A better understanding of the present invention will be given through the following examples and experimental examples, which are not to be construed as limiting the present invention.

### <Examples 1 to 3 - Preparation of *Azadirachta indica* leaf extract>

*Azadirachta indica* leaves used in Examples of the present invention were purchased from TNHH SBJ Viet Nam Co.

The hot-water extract (AIW) of Example 1 was obtained in a manner in which a dried sample was extracted three times at 100°C for 4 hr using, as a solvent, ultrapure water in an amount corresponding to 10 times the weight of the dried sample to afford an extract solution, which was then filtered using filter paper (No. 20, Hyundai Micro Co., Ltd., Korea), concentrated, freeze-dried, and then powdered.

Each of the 70% ethanol extract (AIE) of Example 2 and the 70% acetone extract (AIA) of Example 3 was obtained in a manner in which a dried sample was extracted through immersion three times at room temperature for 24 hr using a solvent in an amount corresponding to 10 times the weight of the dried sample to afford an extract solution, which was then treated and powdered in the same manner as in the preparation of the hot-water extract (FIG. 1).

The hot-water extract yield was 16.55%, the 70% ethanol extract yield was 29.32%, and the 70% acetone extract yield was 26.38%. Individual extracts were stored in a refrigerator at 4°C and used as samples in the following Experimental Examples.

### <Experimental Example 1 - Measurement of antioxidant effect>

### (1) Measurement reagent

The reagents used for the measurement of antioxidant effect were as follows.

2,2-azino-bis(3-ethylbenzothiazoline-6-sulfonic acid)diammonium salt, potassium peroxodisulfate, butylated hydroxy anisole (BHA), L-ascorbic acid, tannic acid, epigallocatechin gallate (ECGC), 2,2-diphenyl-1-picrylhydrazyl, pyrogallol, 2,6-dihydroxypurine, nitro blue tetrazolium, xanthine oxidase and the like were purchased from Sigma Aldrich Co., Ltd. (St. Louis, MO, USA). A Folin-Ciocalteu phenol reagent was purchased from Junsei Chemical Co., Ltd. (Japan). Ethanol, 2-amino-2-(hydroxymethyl)propane-1,3-diol, hydrogen chloride, potassium phosphate monobasic, potassium phosphate dibasic and the like were purchased from Duksan Pure Chemicals Co., Ltd. (Korea).

In each experiment, a sample was dissolved at 10 mg/mL in distilled water and then diluted at concentrations of 5, 10, 50, 100, 500, and 1000 μg/mL.

**(2) Measurement of total polyphenol compound content**

The total phenol compound content was measured through a Folin-Denis method. Each sample was dissolved at 10 mg/mL in distilled water and then diluted at concentrations of 5, 10, 50, 100, 500, and 1000 μg/mL. 0.2 mL of a Folin-Ciocalteu reagent was added thereto and mixed well, and the resulting mixture was then allowed to stand at room temperature for 3 min. After 3 min, 0.5 mL of a 0.7 M $Na_2CO_3$ saturated solution was added thereto and mixed therewith, after which 0.5 mL of each extract was added at different concentrations, followed by reaction for 1 hr and then measurement of absorbance at 725 nm. The polyphenol content was measured from the standard curve using tannic acid.

The phenolic fractions are contained in large amounts in natural substances, and the main function thereof is reported to be the scavenging of free radicals. Also, phenolic fractions such as flavonoids or phenolic acids and anthocyanins act as important factors for antioxidant activity, such as DPPH radical scavenging ability. In the present experiment, the polyphenol content of the *Azadirachta indica* extract was measured from the standard curve using tannic acid as a fundamental material. The results thereof are shown in Table 1 below.

[Table 1]

| Solvent type extract | Polyphenol contents (mg/g)[1] |
|---|---|
| Example 1(Water) | 52,94 ± 0.96[2] |
| Example 2(70% Ethanol) | 51.37 + 0.60[2] |
| Example 3(70% Acetone) | 73.45 ± 0.27[2] |
| [1] TAE standards for tannic acid equivalents.<br>[2] All Value are expressed as Mean ± SD of triplicate determinations.<br>[3] Different superscripts within the column are significantly different at *p*<0.05 by Duncan's multiple range test. | |

As is apparent from Table 1, the polyphenol content was 52.94 mg/g in the *Azadirachta indica* hot-water extract (AIW), 51.37 mg/g in the 70% ethanol extract (AIE), and 73.45 mg/g in the 70% acetone extract (AIA), based on which AIA, among the solvent extracts, was found to have the highest polyphenol content. When compared with Pueraria flower (6.46 mg/g), Pueraria root (5.50 mg/g) and wild chrysanthemum flower (2.48 mg/g) in polyphenol content analysis of medicinal plant extracts (Shin JH, Jang JR, Kang MJ, Shin JH (2018) Physiological Activity of Five Kinds of Medicinal Plant Extracts with Various Solvents and Their Composites. Journal of Life Science, 28, 3, 320-330), it was confirmed that AIW, AIE, and AIA had high polyphenol content.

**(3) Measurement of DPPH radical scavenging effect**

The DPPH radical scavenging ability was measured through a Blois method. 2,2-diphenyl-1-picrylhydrazyl (DPPH) was dissolved to a concentration of 0.45 mM in 99% ethanol, and each extract was diluted at concentrations of 5, 10, 50, 100, 500, and 1000 μg/mL. As a positive control, butylated hydroxyanisole (BHA) was used. Thereafter, 120 μL of each extract and 60 μL of DPPH were added to a 96-well plate and allowed to react in the dark at room temperature for 15 min, and the scavenging ability was measured using an ELISA reader at 517 nm.

$$DPPH\ radical\ scavenging\ ability\ (\%) = (1 - \frac{absorbance\ of\ sample - added\ group}{absorbance\ of\ non - added\ group}) \times 100$$

1,1-diphenyl-2-picrylhydrazyl (DPPH) is used for the measurement of electron-donating ability, and the material itself has a very stable free radical and is a purple fraction showing light absorption that is distinguished from other wavelengths at 517 nm. DPPH is stable in organic solvents such as alcohols and is decolored by a proton-radical scavenger during the antioxidant mechanism and thus the antioxidant activity is easy to visually identify. The antioxidant efficacy of the *Azadirachta indica* leaf extract was determined by measuring the DPPH radical scavenging ability, and the results thereof are shown in FIG. 2a.

As shown in FIG. 2a, all of AIW, AIE, and AIA exhibited concentration-dependent antioxidant effects, and in particular, AIW exhibited the greatest activity at a given concentration. AIW showed an effect of 41.41% at a concentration of 1,000 μg/mL, and AIE and AIA exhibited 34.09% and 30.61%, respectively, indicating that AIW was found to be more effective than the other extracts.

**(4) Measurement of ABTS⁺ radical scavenging effect**

The antioxidant activity using ABTS radicals was measured according to an ABTS⁺ cation decolorization assay method. 7 mM 2,2-azino-bis and 2.4 mM potassium persulfate were mixed in equal amounts and allowed to stand and react in the dark for 12 to 24 hr, after which the experiment was conducted. Each extract was diluted at concentrations of 5, 10, 50, 100, 500, and 1000 μg/mL, and as a positive control, butylated hydroxyanisole (BHA) was used. Thereafter, 100 μL of an ABTS⁺ solution and 100 μL of each extract were added to a 96-well plate and allowed to react at 25°C for 7 min, and absorbance was measured at a wavelength of 734 nm and represented as an inhibition rate (%).

$$ABTS^+ \ radical \ scavenging \ ability \ (\%) = (1 - \frac{absorbance \ of \ sample - added \ group}{absorbance \ of \ non - added \ group}) \times 100$$

With regard to the ABTS radical scavenging ability, ABTS (2,2'-azino-bis(3-ethylbenzothiazoline-6-sulfonic acid)) reacts with potassium persulfate to produce an ABTS⁺ radical having a distinctive turquoise color, which then turns light green when added to a sample, and has the characteristic of measuring both a hydrogen-donating antioxidant and a chain-breaking antioxidant. It is used in many studies along with the DPPH scavenging ability test method because the absorbance changes through reaction with antioxidants. The antioxidant efficacy of the *Azadirachta indica* leaf extract was determined by measuring the ABTS cation radical scavenging ability, and the results thereof are shown in FIG. 2b.

As shown in FIG. 2b, at the highest concentration of 1,000 μg/mL, AIW exhibited an inhibition rate of 73.55%, and AIE and AIA exhibited inhibition rates of 69.60% and 62.24%, respectively. It was confirmed that AIW exhibited the highest ABTS⁺ cation radical scavenging ability at a given concentration.

**(5) Measurement of superoxide-dismutase-like activity**

Superoxide-dismutase-(SOD)-like activity was measured through a Marklund method. Each sample was diluted at concentrations of 5, 10, 50, 100, 500, and 1000 μg/mL, and as a positive control, L-ascorbic acid was used. 20 μL of each sample solution, 130 μL of 50 mM Tris-HCl buffer (pH 8.5), and 20 μL of 7.2 mM pyrogallol dissolved in distilled water were added to a 96-well plate and allowed to react in the dark at room temperature for 20 min, after which absorbance was measured at 420 nm, thus determining the amount of pyrogallol.

$$SOD - like \ activity \ (\%) = (1 - \frac{absorbance \ of \ sample - added \ group}{absorbance \ of \ non - added \ group}) \times 100$$

Superoxide dismutase (SOD) is an enzyme that generates hydrogen peroxide ($H_2O_2$) by reacting with a harmful superoxide anion radical ($\cdot O_2$) *in vivo*, is present in all living organisms that use oxygen, and is a representative inhibitor that acts as a defense against reactive oxygen species *in vivo*. It is also an enzyme ($2O_2^- + 2H^+ \rightarrow H_2O_2 + O_2^-$) involved in a defense mechanism that removes superoxide anion radicals ($\cdot O_2^- \cdot 2O_2 + 2e^- \cdot 2 \cdot O_2^-$) resulting from reduction of oxygen molecules, and is used as an additive for cosmetics, such as an anti-inflammatory material or a cosmetic material for preventing skin aging by acting to prevent the generation of hydroxy radicals having very strong toxicity. The antioxidant effect of the *Azadirachta indica* leaf extract was determined by measuring the effect of the *Azadirachta indica* leaf extract on SOD-like activity, and the results thereof are shown in FIG. 2c.

As shown in FIG. 2c, AIW, AIE and AIA respectively exhibited effects of 12.82%, 24.13% and 7.38% at the highest concentration of 1,000 μg/mL, indicating that the effect was strongest in the order of AIE > AIW > AIA.

**(6) Measurement of superoxide anion radical scavenging effect**

Superoxide anion radical scavenging ability was measured through a nitroblue tetrazolium (NBT) reduction method. Each sample was diluted at concentrations of 5, 10, 50, 100, 500, and 1000 μg/mL, and 0.1 mL of each sample was added with 2 mL of a substrate containing NBT (0.24 mM) and xanthine (0.4 mM) dissolved in 0.4 mL of a 0.1 M potassium phosphate buffer (pH 7.5), further added with 2 mL of xanthine oxidase (0.2 U/mL), allowed to react at 37°C for 20 min, and added with 1 mL of 1 N HCl, and absorbance was measured at 560 nm. As a positive control, epigallocatechin gallate (EGCG) was used.

$$Inhibitory \quad activity \quad (\%) \; = \; (1 - \frac{absorbance \quad of \quad sample \; - \; added \quad group}{absorbance \quad of \quad non - added \quad group}) \times 100$$

A superoxide anion radical is a radical in which very strong toxicity is generated in the enzymatic or non-enzymatic reactions of aerobic cells and is involved in the initial stages of oxidation reactions, which are associated with aging. It is also known to induce oxidative damage to lipids, DNA, proteins, etc. by participating in the generation of reactive oxygen species such as hydrogen peroxide, hydroxy radicals, singlet oxygen, etc. During this process, when the superoxide anion radical reacts with the antioxidant and is thus scavenged, the antioxidant effect is measured using the change in absorbance due to the reduction in the phenomenon of purple color development. The antioxidant effect of the *Azadirachta indica* leaf extract was determined by measuring the superoxide anion radical scavenging ability, and the results thereof are shown in FIG. 2d.

As shown in FIG. 2d, at the highest concentration of 1,000 μg/mL, AIW, AIE and AIA exhibited efficiencies of 81.37%, 96.04% and 52.30%, respectively. At a given concentration, AIE showed the highest efficacy, and the superoxide anion radical scavenging ability was greater for AIW than for AIA.

**<Experimental Example 2 - Measurement of antibacterial effect>**

**(1) Strain and medium**

The strains and media used for the measurement of antibacterial effect were as follows.

As strains, *Escherichia coli, Staphylococcus aureus, Staphylococcus epidermidis, Pseudomonas aeruginosa, Enterobacter cloacae, Propionibacterium acnes, Candida albicans,* and the like were used. As media, nutrient broth (NB), tryptic soy broth (TSB), Gifu anaerobic medium (GAM), nutrient agar (NA), tryptic soy agar (TSA), and the like were used.

(2) Bacterial culture

The strains used for the antibacterial activity measurement experiment were *Escherichia coli* KCTC 2441, *Staphylococcus aureus* KCTC 1916, *Enterobacter cloacae subsp. cloacae* KCTC 2361, *Staphylococcus epidermis* KCTC 1917, and *Pseudomonas aeruginosa* KCTC 1750, and *Propionibacterium acnes* KCTC 3065 was used as an acne-causing bacterium. As a fungus, *Candida albicans* KCTC 7965 was subcultured and used in the experiment. As for liquid media for pretreatment culture and culture in the present experiment, the liquid medium for *Escherichia coli, Staphylococcus epidermis, Pseudomonas aeruginosa* and *Enterobacter cloacae subsp. cloacae* was nutrient broth (NB) purchased from Difco Laboratories (Sparks, USA), the liquid medium for *Staphylococcus aureus* was tryptic soy broth (TSB) purchased from Difco Laboratories (Sparks, USA), the liquid medium for *Propionibacterium acnes* was Gifu anaerobic medium (GAM, Nissui Co. Japan), and the liquid medium for *Candida albicans* was yeast mold broth (YMB, Sparks, USA). As for solid media, nutrient agar (NA), tryptic soy agar (TSA), and yeast mold agar (YMA) were purchased from Difco Laboratories (Sparks, USA), and Gifu anaerobic medium (GAM) was purchased from Nissui Co. Japan. Here, agar powder was added to the present medium and used in the experiment.

**(3) Measurement of inhibitory zone (clear zone)**

The growth inhibition ability was measured using a paper disc. Specifically, each strain cultured in a plate medium was taken with 1 platinum loop, cultured in 15 mL of a liquid medium for 18 to 24 hr, and activated. Thereafter, 0.1 mL of a bacterial solution was seeded in 15 mL of a liquid medium, cultured for 4 to 8 hr, seeded at about $1 \times 10^6$ cells per plate medium and uniformly spread using a sterile swab. Then, a sterilized filter paper disc (8 mm, Tokyo, Japan) was placed on a solid plate medium and the sample was absorbed in an amount of 0.05 mL/disc at different concentrations. Bacteria were cultured in an incubator at 37°C and fungi were cultured in an incubator at 25°C for 12 to 48 hr, and the diameter of the clear zone (mm) around the disc was measured to determine the inhibitory activity.

The antibacterial effect of the *Azadirachta indica* leaf extract was determined by measuring the inhibitory zone (clear zone) through a paper disc agar diffusion assay method, and the results thereof are shown in Table 2 below.

[Table 2]

| Microorganism | Size of clear zone (mm)[1] | | | |
|---|---|---|---|---|
| Micro organism | mg/disc | AIW | AIE | AIA |
| *Enterobacter cloacae* | 1 | -2) | - | - |
| Enterobacter cloace | 2 | - | - | - |
| Enterobacter cloace | 5 | - | - | - |
| Enterobacter cloace | 10 | - | - | - |
| *Staphylococcus aureus* | 1 | - | - | - |
| *Staphylococcus aureus* | 2 | - | 10±0 | 10±0 |
| *Staphylococcus aureus* | 5 | - | 13±0.12 | 12±0 |
| *Staphylococcus aureus* | 10 | - | 16±0.05 | 17±0.15 |
| *Staphylococcus epidermis* | 1 | - | - | - |
| *Staphlococcus epidermis* | 2 | - | - | - |
| *Staphlococcus epidermis* | 5 | - | - | - |
| *Staphlococcus epidermis* | 10 | - | - | - |
| *Pseudomonas aeruginosa* | 1 | - | - | - |
| *Pseudomonas auruginosa* | 2 | - | - | - |
| *Pseudomonas auruginosa* | 5 | - | - | - |
| *Pseudomonas auruginosa* | 10 | - | - | - |
| *Escherichia coli* | 1 | - | - | - |
| *Escherichia coli* | 2 | - | - | - |
| *Escherichia coli* | 5 | - | - | - |
| *Escherichia coli* | 10 | 10±0 | - | - |
| *Propionibacterium acnes* | 1 | - | - | - |
| *Propionibacterium acnes* | 2 | - | - | - |
| *Propionibacterium acnes* | 5 | 11±0 | 13±0.05 | 13±0 |
| *Propionibacterium acnes* | 10 | 14±0 | 15±0 | 15±0 |
| *Candida albicans* | 1 | - | - | - |
| Candidia albicans | 2 | - | - | - |
| Candidia albicans | 5 | - | - | - |
| Candidia albicans | 10 | - | - | - |
| 1) Diameter (Disc 8mm), | | | | |
| 2) No inhibitory zone was formed. The values are mean ± standard deviation of triplicate determination. | | | | |

As is apparent from Table 2, the *Azadirachta indica* leaf extract did not have an antibacterial effect against *Escherichia coli, Staphylococcus epidermidis, Pseudomonas aeruginosa, Enterobacter cloacae,* or *Candida albicans.* Moreover, AIE and AIA showed a clear zone of 1.0±0 cm or more from 2 mg/disc against *Staphylococcus aureus,* and AIW, AIE, and AIA showed a clear zone of 1.1±0 cm or more from 5 mg/disc against *Propionibacterium acnes.*

**<Experimental Example 3 - Measurement of whitening effect>**

**(1) Measurement reagent**

The reagents used for the measurement of whitening effect were as follows.

3,4-dihydroxy-L-phenylalanine, mushroom tyrosinase and the like were purchased from Sigma Aldrich Co., Ltd. (St. Louis, MO, USA). Sodium phosphate monobasic dihydrate, sodium phosphate dibasic anhydrous and the like were purchased from Duksan Pure Chemicals Co., Ltd. (Korea). MITF, tyrosinase, TRP-1, and TRP-2 secondary antibodies used to measure the whitening effect were purchased from Santa Cruz (Biotech, CA, USA).

(2) Measurement of mushroom-derived tyrosinase inhibitory activity

The tyrosinase inhibitory activity was measured according to the method of Yagi *et al.* Each sample was diluted at concentrations of 5, 10, 50, 100, 500, and 1000 μg/mL, and as a positive control, L-ascorbic acid was used. 40 μL of mushroom tyrosinase [125 unit/mL] was added to a mixed solution of 40 μL of the sample and 40 μL of a substrate containing 10 mM L-DOPA dissolved in 80 μL of a 67 mM sodium phosphate buffer (pH 6.8) and allowed to react at 37°C for 10 min, after which absorbance was measured at 492 nm.

$$Tyrosinase\ inhibitory\ activity\ (\%) = (1 - \frac{absorbance\ of\ sample - added\ group}{absorbance\ of\ non - added\ group}) \times 100$$

Melanocytes are biosynthesized in the melanosome in the pigment cells of the basal layer of the epidermis, in which melanin acts as an important factor in determining the skin tone. Tyrosine, which is a kind of amino acid, is a material that synthesizes melanin. Tyrosine is oxidized into L-3,4-dihydroxyphenylalanine (DOPA) and DOPA quinone by tyrosinase in melanoma cells. Thereafter, the DOPA quinone is converted into 5,6-dihydroxyindole, DOPA chrome, and indole-5,6-quinone, thus producing melanin by polymerization of indole-5,6-quinone. In order to measure the inhibitory activity of tyrosinase, which is able to inhibit the biosynthesis of melanin polymer in the skin, the mushroom-derived tyrosinase inhibitory activity was measured, and the results thereof are shown in FIG. 3a.

As shown in FIG. 3a, at the highest concentration of 1,000 μg/mL, AIW exhibited 32.77%, AIE exhibited 47.21%, and AIA exhibited 45.36%, and at a given concentration, AIE exhibited the highest activity (in which the data represents the mean ± SD of three separate experiments). Although having a lower effect than L-ascorbic acid used as a positive control, the *Azadirachta indica* leaf extract was confirmed to have a significant effect as the natural substance and is deemed to be useful in whitening.

**<Experimental Example 4 - Measurement of wrinkle reduction effect>**

**(1) Measurement reagent**

The reagents used for the measurement of wrinkle reduction effect were as follows.

N-succinyl-Ala-Ala-Ala-p-nitroanilide, elastase from porcine pancreas, and the like were purchased from Sigma Aldrich Co., Ltd. (St. Louis, MO, USA). 2-amino-2-(hydroxymethyl)propane-1,3-diol, hydrogen chloride, and the like were purchased from Duksan Pure Chemicals Co., Ltd. (Korea). MMP-1 and filaggrin secondary antibodies were purchased from Santa Cruz (Biotech, CA, USA).

**(2) Measurement of elastase inhibition effect**

Elastase inhibitory activity was measured with reference to the method of Cannell *et al.* Using N-succinyl-(L-Ala)$_3$-p-nitroanilide, the amount of p-nitroanilide produced from the substrate at 36°C for 25 min was measured by ELISA at 405 nm. Thereafter, each sample was diluted at concentrations of 5, 10, 50, 100, 500, and 1000 μg/mL, and 40 μL of each sample solution was added with 40 μL of a porcine pancreas elastase [0.5 unit/mL] solution dissolved in a 50 nM tris-HCl buffer (pH 8.6), added with N-succinyl-(L-Ala)$_3$-p-nitroanilide dissolved in a 50 mM tris buffer (pH 8.6) as a substrate, and allowed to react for 20 min, after which the results were measured. As a positive control, epigallocatechin gallate (EGCG) was used.

$$Elastase\ inhibitory\ activity\ (\%) = (1 - \frac{absorbance\ of\ sample - added\ group}{absorbance\ of\ non - added\ group}) \times 100$$

Elastin is a very important protein for maintaining skin elasticity in the dermis, is present in the connective tissue of the skin, and is involved in tissue elasticity and flexibility. Elastase is an enzyme that degrades elastin and is regarded as the main cause of skin aging, such as wrinkling and skin sagging. Therefore, elastase inhibitors act to prevent aging through skin wrinkle reduction. The effect of the *Azadirachta indica* leaf extract on elastase inhibitory activity was determined, and the results thereof are shown in FIG. 3b.

As shown in FIG. 3b, at the highest concentration of 1,000 $\mu$g/mL, AIW, AIE, and AIA exhibited activities of 17.56%, 24.24%, and 21.92%, respectively, among which AIE exhibited the highest activity (in which the data represents the mean $\pm$ SD of three separate experiments).

## <Experimental Example 5 - Measurement of anti-inflammatory effect>

### (1) Cell line and reagent

As cell lines used to measure the cell viability, murine macrophage cells (RAW 264.7) and mouse melanoma cells (B16F10) were purchased from the American Type Culture Collection (Manassas, VA, USA). Human keratinocytes (HacaT) were obtained from the Korean Institute for Promotion of the Traditional Medicine Industry. Dulbecco's Modified Eagle medium (DMEM), fetal bovine serum (FBS) and the like were purchased from LONZA Co. 0.25% trypsin-EDTA and 0.4% trypan blue stain were purchased from Gibco BRL Co. (Grand Island, NY, USA), and a 3-[4,5-dimethylthiazol-2-yl]-2,5-diphenyltetrazolium bromide (MTT) reagent was purchased from Sigma Chemical Co. Ltd. (St. Louis, MO, USA).

### (2) Cytotoxicity measurement

1) Cell culture

RAW 264.7 macrophage cells, B16F10 melanoma cells, and HacaT keratinocytes used for the present experiment were subcultured in a 5% $CO_2$ incubator at 37°C using DMEM containing 1% penicillin/streptomycin (100 unit/mL) and 10% FBS.

2) Measurement of cell viability through MTT assay

The cell viability was measured through a Carmichael method. As cell lines, RAW 264.7, B16F10, and HaccaT cells were aliquoted at 1 x $10^4$ cells/well to a 96-well plate and cultured in a 5% $CO_2$ incubator at 37°C for 24 hr. Each of AIW, AIE, and AIA was diluted at concentrations of 5, 10, 25, 50, 100, 250, and 500 $\mu$g/mL and the cells were treated with 200 $\mu$L/well thereof for 24 hr. Thereafter, the cells were added with 20 $\mu$L/well of an MTT solution prepared at a concentration of 2.5 mg/mL and then cultured for 3 to 4 hr, after which the culture medium was removed, followed by addition with dimethyl sulfoxide (DMSO) in an amount of 100 $\mu$L/well and reaction at room temperature for 10 min, after which absorbance was measured at 540 nm.

$$Cell\ viability\ (\%) = (1 - \frac{absorbance\ of\ sample - added\ group}{absorbance\ of\ non - added\ group}) \times 100$$

The macrophage cell viability was measured using *Azadirachta indica* through an MTT assay, and the results thereof are shown in FIG. 4a.

As shown in FIG. 4a, based on the results of measurement at different concentrations, all of AIW, AIE, and AIA exhibited cell viability of 90% or more at concentrations of 5, 10, 50, and 100 $\mu$g/mL, and Western blot and real-time PCR were performed in three sections of 10, 50, and 100 $\mu$g/mL (in which C is the group treated with LPS. The data represent the mean $\pm$ SD of three separate experiments. The difference with the control was found to be significant. * $p<0.05$, ** $p<0.01$, *** $p<0.005$).

### (3) Measurement of nitric oxide generation inhibition effect

The amount of nitric oxide (NO) in the supernatant of the cells was measured based on nitrite and nitrate. Cell line RAW 264.7 was aliquoted at 4 x $10^4$ cells/well to a 96-well plate and cultured in a 5% $CO_2$ incubator at 37°C for 24 hr. Thereafter, the culture medium was replaced with serum-free DMEM and an inflammatory response was induced using lipopolysaccharide (LPS). After treatment with LPS at 1 ppm/well, the cells were treated with each sample solution at different concentrations and then cultured in a 5% $CO_2$ incubator at 37°C for 24 hr. Then, 100 $\mu$L of the cell culture solution was transferred to a new 96-well plate, added with 100 $\mu$L of Griess' reagent, and allowed to react at room temperature for 5 min, after which absorbance was measured at 540 nm and the results were calculated.

$$NO \ generation \ amount \ (\%) = (1 - \frac{absorbance \ of \ sample - added \ group}{absorbance \ of \ non - added \ group}) \times 100$$

In order to determine the extent of nitric oxide inhibition of chitosan-gluconate in the macrophage cell RAW 264.7 by *Azadirachta indica,* the NO amount was measured by treating samples using different solvents and at different concentrations, and the results thereof are shown in FIG. 4b.

As shown in FIG. 4b, at the highest concentration of 100 $\mu$g/mL, AIW, AIE, and AIA exhibited inhibition effects of 35.7%, 68.86%, and 66.45%, respectively. At a given concentration, AIE exhibited the greatest NO inhibitory activity (in which N is the group not treated with LPS, and C is the group treated with LPS. The data represents the mean $\pm$ SD of three separate experiments. The difference with the control was found to be significant. * $p<0.05$, ** $p<0.01$, *** $p<0.005$).

### (4) Measurement of protein expression through Western blot

In order to evaluate the expression of iNOS, COX-2, tyrosinase, TRP-1, TRP-2, MMP-1, and filaggrin protein, as cell lines, RAW 264.7 and HacaT cells were aliquoted at 3 x $10^5$ cells/well to a 6-well plate, and B16F10 was aliquoted at 2 x $10^5$ cells/well, followed by culture in a 5% $CO_2$ incubator at 37°C for 24 hr. The culture medium was replaced with serum-free DMEM, after which, in order to induce cell inflammation, whitening and wrinkle reduction, RAW 264.7 and B16F10 were treated with lipopolysaccharide (LPS) and alpha-melanocyte-stimulating hormone ($\alpha$-MSH), respectively, at 1 ppm/well, and the cells were treated with each sample solution at different concentrations and cultured in a 5% $CO_2$ incubator at 37°C for 24 hr. As for HacaT cells, all experimental groups except the untreated group were washed once with PBS, added with PBS, and irradiated with UV-B (300 mJ/cm$^2$). After irradiation, the cells were treated with the sample solution at different concentrations and cultured for 24 hr to 48 hr. The medium was then removed, after which the cells were washed once with PBS, treated with 60 $\mu$L/well of a RIPA buffer containing 0.1% phosphatase and a 0.1% protease inhibitor, lysed, and centrifuged at 4°C and 12,000 rpm for 15 min, and the resulting supernatant was transferred into a new tube. The membrane was blocked in the order of 5% skim milk for 2 hr, primary antibody for 2 hr, 1x TBST solution for 10 min (repeat 3 times), secondary antibody for 1 hr, and 1x TBST solution for 10 min (repeat 3 times) using a digital reciprocating shaker, followed by reaction for 2 min using an HRP substrate color development reagent and then measurement of bands using a western imaging system (CAS-400SM, Davinch-K Co., Ltd., Korea). The quantitative results thereof were calculated.

In order to evaluate the effect of *Azadirachta indica* on iNOS and COX-2, LPS-induced mouse macrophage cells (RAW 264.7) were treated with AIW, AIE, and AIA at concentrations of 10, 50, and 100 $\mu$g/mL for 24 hr and then recovered, after which the expression thereof was measured through Western blot. As a control, ammonium pyrrolidinedithiocarbamate (PDTC) was used. As a housekeeping gene, $\beta$-actin was used. The results of AIW, AIE and AIA are shown in FIGS. 5a, 5b and 5c, respectively.

As shown in FIG. 5a, 5b and 5c, AIE inhibited the expression of iNOS, and AIA inhibited the expression of iNOS and COX-2 protein (in which N is the group not treated with LPS, and C is the group treated with LPS. The data represents the mean $\pm$ SD of three separate experiments. The difference with the control was found to be significant. * $p<0.05$, ** $p<0.01$, *** $p<0.005$).

### (5) Isolation of mRNA and real-time PCR analysis

As cell lines, RAW 264.7, B16F10 and HacaT cells were aliquoted at 3 x $10^5$ cells/well to a 6-well plate and cultured in a 5% $CO_2$ incubator at 37°C for 24 hr. Then, the culture medium was replaced with serum-free DMEM, after which, in order to induce cell inflammation, whitening and wrinkle reduction, RAW 264.7 and B16F10 were treated with lipopolysaccharide (LPS) and alpha-melanocyte-stimulating hormone ($\alpha$-MSH), respectively, at 1 ppm/well, and the cells were treated with each sample solution at different concentrations and then cultured in a 5% $CO_2$ incubator at 37°C for 24 hr. As for HacaT cells, all experimental groups except the untreated group were washed once with PBS, added with PBS, and irradiated with UV-B (300 mJ/cm$^2$). After irradiation, the cells were treated with the sample solution at different concentrations and cultured for 24 hr to 48 hr. The medium was then removed, after which the cells were washed once with PBS, added with 1 mL/well of a TRI-solution, lysed, transferred into a new tube, and cultured at room temperature for 5 min. Thereafter, the cells were added with 200 ml of chloroform per tube, strongly vortexed, cultured for 5 min, and centrifuged at 4°C and 12000 rpm for 20 min, and 500 $\mu$L of the clear supernatant was transferred into a new tube. The supernatant was added with 500 $\mu$L of isopropyl alcohol, inverted several times, cultured for 5 min, and centrifuged at 4°C and 12000 rpm for 20 min, and the supernatant was then removed. The pellets that precipitated to the bottom were washed with 1 mL of 75% ethanol, completely dried, and then lysed with 50 $\mu$L of DEPC water. Thereafter, RNA was quantified using absorbance, and cDNA was then synthesized using a TProfessional basic thermocycler instrument. Thereafter, 40 PCR cycles of 95°C for 10 sec, 60°C for 15 sec, and 72°C for 20 sec were performed using Power SYBR Green PCR Master Mix reagents, primers and cDNA and using a Step One real-time PCR system, and quantitative analysis was conducted using an analysis program. The sequences used for the experiment are shown in Table 3 below.

[Table 3]

| Gene | Primer | Sequence (5'→3') |
|---|---|---|
| iNOS | Sense | ACA TCG ACC CGT CCA CAG TAT |
| iNOS | Antisense | CAG AGG GGT AGG CTT GTC TC |
| TRP-1 | Sense | CCC CTA GCC TAT ATC TCC CTT TT |
| TRP-1 | Antisense | TAC CAT CGT GGG GAT AAT GGC |
| GAPDH | Sense | TGA CCA CAG TCC ATG CCA TC |
| GAPDH | Antisense | GAC GGA CAC ATT GGG GGT AG |

Real-time PCR is widely used because it enables faster measurement than conventional PCR and is relatively non-pollutive. Based on the results of the above Western blot and antioxidant experiments, it was confirmed that AIE was the most effective, and thus AIE was selected for the present experiment. The results of measurement of the inhibition rate of iNOS mRNA expression using AIE through real-time PCR are shown in FIG. 6a.

As shown in FIG. 6a, the effect of AIE on inhibiting iNOS expression was found to be 97.49% at the highest concentration of 100 $\mu$g/mL (in which N is the group not treated with LPS, and C is the group treated with LPS. The data represents the mean $\pm$ SD of three separate experiments. Compared to the LPS-treated control, * $p < 0.01$ represents a significant difference).

**<Experimental Example 6 - Measurement of whitening efficacy of *Azadirachta indica* leaf extract>**

(1) Evaluation of cytotoxicity of melanoma cells (B16F10)

The results of evaluation of melanoma cell viability using *Azadirachta indica* (70% ethanol extraction) through an MTT assay are shown in FIG. 6b.

As shown in FIG. 6b, AIE exhibited cell viability of 85% or more at all concentrations of 2.5, 5, 10, and 25 $\mu$g/mL, and Western blot was performed in the top three sections (in which N is the group not treated with $\alpha$-MSH. The data represents the mean $\pm$ SD of three separate experiments. The difference with the control was found to be significant. * $p < 0.05$, ** $p < 0.01$).

(2) Evaluation of inhibition of TRP-1, TRP-2 and tyrosinase expression through Western blot

Alpha-melanocyte-stimulating hormone ($\alpha$-MSH), which is involved in melanin biosynthesis, regulates melanin synthesis using the melanocortin-1 receptor (MC1R), which activates the cAMP pathway. When cAMP continues to increase in cells, PKA (cAMP-dependent protein kinase or protein kinase A) phosphorylates CREB (cAMP response element binding protein) to thus increase MITF expression, which induces tyrosinase gene expression. In order to evaluate the effect of *Azadirachta indica* extract on melanin production, factors affecting melanin formation were measured through Western blot. The results thereof are shown in FIG. 6c (in which A: TRP-1, B: TRP-2, C: tyrosinase melanoma cells).

As shown in FIG. 6c, based on the experimental results of inhibition of melanin formation, AIE inhibited TRP-1 by 36.35% at the highest concentration of 10 $\mu$g/mL, induced by $\alpha$-MSH, and did not inhibit the other factors, such as TRP-2 and tyrosinase (in which N is the group not treated with $\alpha$-MSH, and C is the group treated with $\alpha$-MSH. The data represents the mean $\pm$ SD of three separate experiments. The difference with the control was found to be significant. * $p < 0.05$, ** $p < 0.01$).

(3) Evaluation of inhibition of TRP-1 expression through real-time PCR

The inhibition rate of TRP-1 mRNA expression by AIE was determined through real-time PCR. The results thereof are shown in FIG. 6d.

As shown in FIG. 6d, the effect of AIE on inhibiting TRP-1 expression was 87.43% at the highest concentration of 10 $\mu$g/mL, which is evaluated to be higher than 14.86% when using, as the control, arbutin at a concentration of 100 $\mu$g/mL (in which N is the group not treated with $\alpha$-MSH, and C is the group treated with $\alpha$-MSH. The data represents the mean $\pm$ SD of three separate experiments. The difference with the control was found to be significant. * $p<0.05$, ** $p<0.01$, *** $p<0.005$).

### <Experimental Example 7 - Measurement of wrinkle reduction efficacy of *Azadirachta indica* leaf extract>

**(1) Evaluation of cytotoxicity of human keratinocytes (HacaT cells)**

The results of evaluation of the cell viability of human keratinocytes using *Azadirachta indica* (70% ethanol extraction) through an MTT assay are shown in FIG. 7a.

As shown in FIG. 7a, the cell viability thereof was 90% or more at up to 10 $\mu$g/mL among AIE concentrations of 1, 2.5, 5, 10, and 25 $\mu$g/mL, and Western blot was performed in three sections of 2.5, 5, and 10 $\mu$g/mL (in which C is the group treated with DMEM alone. The data represents the mean $\pm$ SD of three separate experiments. The difference with the control was found to be significant. * $p<0.05$, ** $p<0.01$).

**(2) Evaluation of MMP-1 and filaggrin protein expression through Western blot**

MMPs are secreted from many skin cells, including fibroblasts and keratinocytes, and about 20 types thereof are known to date. MMP-1 is known as collagenase 1, and uses collagen type I and III matrices. A precursor called profilaggrin accumulates within cytoplasmic bodies known as keratohyalin granules, which are a prominent feature of keratinocytes. In the present experiment, the expression of MMP-1 and filaggrin protein was measured, and the results thereof are shown in FIG. 7b.

As shown in FIG. 7b, AIE inhibited MMP-1 by 9.93% at the highest concentration of 10 $\mu$g/mL, and had a moisturizing effect of 32.52% in filaggrin (in which N is the group not treated with UV-B, and C is the group treated with UV-B. The data represents the mean $\pm$ SD of three separate experiments. The difference with the control was found to be significant. * $p<0.05$, ** $p<0.01$).

### <Experimental Example 8> Measurement of anti-pollution efficacy of *Azadirachta indica* leaf extract

**(1) Evaluation of cytotoxicity**

The results of evaluation of the cell viability of keratinocytes (HaCaT cells) and fibroblasts (CCD-986sk cells) using *Azadirachta indica* (70% ethanol extraction) through an MTT assay are shown in FIGS. 8a and 8b.

As shown in FIG. 8a, the cell viability of keratinocytes (HaCaT cells) was 90% or more at AIE concentrations of 5, 10, 25, 50, and 100 $\mu$g/mL (in which C is the group not treated with UV-B. The data represents the mean $\pm$ SD of three separate experiments. The difference with the control was found to be significant. * $p<0.05$, ** $p<0.01$).

As shown in FIG. 8b, the cell viability of fibroblasts (CCD-986sk cells) was 90% or more at AIE concentrations of 5, 10, 25, 50, and 100 $\mu$g/mL (in which C is the group treated with DMEM alone. The data represents the mean $\pm$ SD of three separate experiments. The difference with the control was found to be significant. * $p<0.05$, ** $p<0.01$).

**(2) Evaluation of anti-pollution efficacy by skin cell protection**

The anti-pollution effect of the *Azadirachta indica* 70% ethanol extract on HaCaT cells as keratinocytes and CCD-986sk cells as fibroblasts was evaluated.

1) HaCaT cells were aliquoted at 1 x $10^4$ cells/well and cultured in a 5% $CO_2$ incubator at 37°C for 24 hr. Then, the culture medium was replaced with serum-free DMEM, followed by stimulation with benzo[$\alpha$]pyrene (BP). Thus, the cells were treated with benzo[$\alpha$]pyrene (BP) at 100 ppm/well, treated with each sample solution at different concentrations, and then cultured in a 5% $CO_2$ incubator at 37°C for 24 hr. Thereafter, the cells were added with 20 $\mu$L/well of an MTT solution prepared at a concentration of 2.5 mg/mL and cultured for 3 to 4 hr, followed by removal of the culture medium, addition with dimethyl sulfoxide (DMSO) in an amount of 100 $\mu$L/well, and reaction at room temperature for 10 min, after which absorbance was measured at 540 nm.

2) For CCD-986sk cells, an anti-pollution experiment on BP was performed in the same manner as above, and an anti-pollution experiment was performed using 150 ppm/well of fine dust in lieu of BP.

The results thereof are shown in FIG. 9.

As shown in FIG. 9a, when the cells were treated with benzo[α]pyrene alone, about 30% thereof were killed, and when the cells treated with benzo[α]pyrene was treated with the *Azadirachta indica* extract at 5, 10, 25, 50, and 100 μg/mL, an improvement effect of 90% or more was exhibited at 25 μg/mL or more compared to the cells treated with benzo[α]pyrene alone (in which N is the group not treated with benzopyrene, and C is the group treated with benzopyrene alone. The data represents the mean ± SD of three separate experiments. The difference with the control was found to be significant. * $p < 0.05$, ** $p < 0.01$).

As shown in FIG. 9b, when CCD-986sk cells were treated with benzo[α]pyrene alone, about 31% thereof were killed, and when the cells treated with benzo[α]pyrene were treated with the *Azadirachta indica* extract at 5, 10, 25, 50, and 100 μg/mL, an improvement effect of 80% or more was exhibited at 25 μg/mL or more compared to the cells treated with benzo[α]pyrene alone (in which N is the group not treated with benzopyrene, and C is the group treated with benzopyrene alone. The data represents the mean ± SD of three separate experiments. The difference with the control was found to be significant. * $p < 0.05$, ** $p < 0.01$).

As shown in FIG. 9c, when CCD-986sk cells were treated with fine dust alone, about 23% thereof were killed, and when the cells treated with fine dust were treated with the *Azadirachta indica* extract at 5, 10, 25, 50, and 100 μg/mL, an improvement effect of 85% or more was exhibited at 100 μg/mL compared to the cells treated with fine dust alone (in which N is the group not treated with fine dust, and C is the group treated with fine dust alone. The data represents the mean ± SD of three separate experiments. The difference with the control was found to be significant. * $p < 0.05$, ** $p < 0.01$).

(3) **Evaluation of anti-pollution efficacy due to prevention of degradation of hyaluronic acid**

In order to evaluate whether the *Azadirachta indica* 70% ethanol extract is able to protect hyaluronic acid, involved in skin moisturization, from heavy metals, iron (325 mg), 0.5 g of *Azadirachta indica* 70% ethanol extract and EDTA-2Na were allowed to react.

[Table 4]

| Sample | Viscosity (cps) |
|---|---|
| Hyaluronic acid | 10,000 cp |
| Hyaluronic acid + iron 10 ppm | 6,800 cp |
| Hyaluronic acid + 1% *Azadirachta indica* extract + iron 10 ppm | 8,200 cp |
| Hyaluronic acid + 0.2% EDTA-2Na + iron 10 ppm | 7,800 cp |

The effect of preventing the degradation of hyaluronic acid was evaluated by measuring the viscosity of hyaluronic acid, and the results thereof are shown in Table 4. When hyaluronic acid is degraded by heavy metals, the viscosity of the solution decreases, so the lower the viscosity, the greater the degradation of hyaluronic acid. As is apparent from Table 4, when compared with EDTA-2Na, which is a complex-forming substance generally used for adsorption of heavy metals, it was confirmed that the effect of binding of the *Azadirachta indica* extract to iron was better and thus the viscosity was higher. Thereby, it can be confirmed for the *Azadirachta indica* extract that the hyaluronic acid degradation prevention effect is further increased.

**<Experimental Example 9 - Preparation of Pickering emulsion and measurement of stability thereof>**

**(1) Materials for Pickering emulsion**

As the powder phase of the Pickering emulsion formulation, uncoated silica (silica silylate, CABOT) was used. As the water phase thereof, moisturizers such as glycerin (vegetable glycerin, LG H&H), 1,3-BG (1,3-butylene glycol, Cosnet), and propanediol (1,3-propanediol, USA) were used. The antioxidant was vitamin C (L-ascorbic acid, DAEJUNG). The preservative was hexanediol (1,2-hexanediol, K1 Solution) and ethylhexylglycerin (K1 Solution).

**(2) Preparation of Pickering emulsion**

In order to prepare the water-in-air emulsion (W/A emulsion), the water phase was weighed as shown in Table 5 below, and the powder-phase silica silylate was placed in a grinder, pulverized at 15,500 rpm for 1 min, transferred into a beaker, and treated using an Agi mixer at 500 rpm for 3 min, thereby preparing a Pickering emulsion in a powder essence form (FIG. 10).

[Table 5]

| Material Name | | Content (wt%) |
|---|---|---|
| Water phase | Water | 53.4 |
| | Moisturizers (Glycerin / 1,3-BG / Propanediol) | 30.0 |
| | L-ascorbic acid | 10.0 |
| Additive phase | Silica silylate | 5.00 |
| | *Azadirachta indica* (AIE, 10% solution) | 1.00 |
| | 1,2-hexanediol | 0.30 |
| | L-arginine | 0.30 |
| | Total | 100 |

The Pickering emulsion of the present Experimental Example was prepared by applying the optimal amounts of moisturizers and L-ascorbic acid and controlling the pH. The Pickering emulsion is configured such that the silica component is surrounded by the water phase, thus blocking air and further increasing stability. Therefore, it can be found that the amounts of the composite moisturizer and the L-ascorbic acid in the Pickering emulsion play an important role in oxidation stability. In the following Experimental Example, the formation of liquid-crystal emulsion through the mixing of materials was confirmed.

**(3) Formulation change depending on amount of moisturizer (glycerin)**

Powder formation was evaluated depending on the amount of glycerin used as a moisturizer. As is apparent from Table 6 below, the results of the change in the formulation when increasing the amount of glycerin to 70% in increments of 10% are shown in FIG. 11a. As shown in FIG. 11a, even when glycerin was added up to 70% for powder formation, it was confirmed that the formulation was good.

[Table 6]

| Phase | Material Name | Content (wt%) | | | | | | | |
|---|---|---|---|---|---|---|---|---|---|
| | | A | B | C | D | E | F | G | H |
| Water phase | Water | to 100 | to 100 | to 100 | to 100 | to 100 | to 100 | to 100 | to 100 |
| | Glycerin | 0 | 10 | 20 | 30 | 40 | 50 | 60 | 70 |
| | 1,3-BG | | | | | | | | |
| | Propanediol | | | | | | | | |
| Additive phase | Silica silylate | 5 | 5 | 5 | 5 | 5 | 5 | 5 | 5 |
| | Total | 100 | 100 | 100 | 100 | 100 | 100 | 100 | 100 |

**(4) Formulation change depending on amount of moisturizer (1,3-butylene glycol)**

The powder formation was evaluated depending on the amount of 1,3-butylene glycol (1,3-BG) used as a moisturizer. As is apparent from Table 7 below, the results of the change in the formulation when increasing the amount of 1,3-BG to 40% in increments of 10% are shown in FIG. 11b. As shown in FIG. 11b, even when 1,3-BG was added up to 30% for powder formation, it was confirmed that the formulation was good.

[Table 7]

| Phase | Material Name | Content (wt%) | | | |
|---|---|---|---|---|---|
| | | A | B | C | D |
| Water phase | Water | to 100 | to 100 | to 100 | to 100 |
| | Glycerin | | | | |
| | 1,3-BG | 10 | 20 | 30 | 40 |
| | Propanediol | | | | |
| Additive phase | Silica silylate | 5 | 5 | 5 | 5 |
| | Total | 100 | 100 | 100 | 100 |

**(5) Formulation change depending on amount of moisturizer (propanediol)**

The powder formation was evaluated depending on the amount of propanediol used as a moisturizer. As is apparent from Table 8 below, the results of the change in the formulation when increasing the amount of propanediol to 50% in increments of 10% are shown in FIG. 11c. As shown in FIG. 11c, even when propanediol was added up to 40% for powder formation, it was confirmed that the formulation was good.

[Table 8]

| Phase | Material Name | Content (wt%) | | | | |
|---|---|---|---|---|---|---|
| | | A | B | C | D | E |
| Water phase | Water | to 100 | to 100 | to 100 | to 100 | to 100 |
| | Glycerin | | | | | |
| | 1,3-BG | | | | | |
| | Propanediol | 10 | 20 | 30 | 40 | 50 |
| Additive phase | Silica silylate | 5 | 5 | 5 | 5 | 5 |
| | Total | 100 | 100 | 100 | 100 | 100 |

**(6) Results of measurement of TEWL depending on amount of composite moisturizer**

After mixing glycerin, 1,3-butylene glycol and propanediol, used as moisturizers, in the amounts shown in Table 9 below, powder formulations A, B, C, and D were evaluated. As shown in FIG. 12, when measuring the difference in transepidermal water loss using a TEWL meter, the water loss after 4 hr was 15.93, 4.06, 18.86 and 12.6 $g/m^2/h$ in A, B, C and D, respectively, which is evaluated to be low compared to water, used as the control. It was confirmed that the composite moisturizer C had the greatest moisturizing capability, and thus the composite moisturizer C was used in the following experiment.

[Table 9]

| Phase | Material Name | Content (wt%) | | | |
|---|---|---|---|---|---|
| | | A | B | C | D |
| Water phase | Water | to 100 | to 100 | to 100 | to 100 |
| | Moisturizers (Glycerin, 1,3-BG, Propanediol) | 30 | 30 | 30 | 30 |
| Additive phase | Silica silylate | 5 | 5 | 5 | 5 |
| | Total | 100 | 100 | 100 | 100 |

**(7) Change in formulation depending on pH**

As is apparent from Table 10 below, the pH of the water phase was adjusted to the range of 3 to 10 using citric acid and L-arginine, and whether the Pickering emulsion formulation of silica was formed depending on the pH was evaluated. The results thereof are shown in FIG. 13a. As shown in FIG. 13a, the emulsion was formed at a pH of 3 to 9, and the formulation adhered to the wall at a pH of 10. Thus, the pH was set to the range of 3 to 9 and the following experiment was performed.

[Table 10]

| Phase | Material Name | Content (wt%) | | | | | | | |
|---|---|---|---|---|---|---|---|---|---|
| | | pH3 | pH4 | pH5 | pH6 | pH7 | pH8 | pH9 | pH10 |
| Water phase | Water | to 100 | to 100 | to 100 | to 100 | to 100 | to 100 | to 100 | to 100 |
| Additive phase | Silica silylate | 5 | 5 | 5 | 5 | 5 | 5 | 5 | 5 |
| | Total | 100 | 100 | 100 | 100 | 100 | 100 | 100 | 100 |

**(8) Change in formulation depending on pH of L-ascorbic acid**

L-ascorbic acid was used in an amount of 1% and the pH of the water phase was measured. As shown in Table 11 below, A, having a pH of 2.98, and B, the pH of which was adjusted to 6.5 using L-arginine, were allowed to stand in a chamber at 45°C for 1 week, and the measurement results thereof are shown in FIG. 13b. As shown in FIG. 13b, B became unstable, and A showed similar results when compared with the first day. Therefore, the following experiment was conducted by adjusting the pH to 2-4 using L-ascorbic acid.

[Table 11]

| Phase | Material Name | Content (wt%) | |
|---|---|---|---|
| | | A | B |
| Water phase | Water | to 100 | to 100 |
| | Moisturizers (Glycerin, 1,3-BG, Propanediol) | 30 | 30 |
| | L-ascorbic acid | 1 | 1 |
| Additive phase | Silica silylate | 5 | 5 |
| | Total | 100 | 100 |
| | pH | 2.95 | 6.5 |

**(9) Change in formulation depending on amount of L-ascorbic acid**

An experiment was conducted in order to evaluate the change in the formulation depending on the amount of L-ascorbic acid (vitamin C). As shown in Table 12 below, when the amount of L-ascorbic acid was increased to 2.00%, 5.00%, 10.00%, and 15.00%, as shown in FIG. 13c, it can be confirmed that the formulation was stable even when L-ascorbic acid was added up to 10% for powder formation.

[Table 12]

| Phase | Material Name | Content (wt%) | | | |
|---|---|---|---|---|---|
| | | A | B | C | D |
| Water phase | Water | to 100 | to 100 | to 100 | to 100 |
| | Moisturizers (Glycerin, 1,3-BG, Propanediol) | 30 | 30 | 30 | 30 |
| | 1,2-hexandiol | 0.3 | 0.3 | 0.3 | 0.3 |
| | L-arginine | 0.3 | 0.3 | 0.3 | 0.3 |
| | L-ascorbic acid | 2 | 5 | 10 | 15 |

(continued)

| Phase | Material Name | Content (wt%) | | | |
|---|---|---|---|---|---|
| | | A | B | C | D |
| Additive phase | Silica silylate | 5 | 5 | 5 | 5 |
| | Total | 100 | 100 | 100 | 100 |

**(10) Measurement of pH depending on temperature and time**

The pH of the skin is determined by measuring the sebaceous layer of the skin, and generally falls in a weakly acidic range. However, if the pH of the skin falls out of the above range, microbes actively proliferate on the skin and may cause irritation, infection, itching, etc. Therefore, the pH of cosmetic products needs to be adjusted and maintained within the above range. In order to measure the change in pH of the Pickering emulsion composition prepared using the composition shown in Table 5, the experiment was performed in the dark at 25°C for 60 days, and the results thereof are shown in Table 13 below.

[Table 13]

| Pickering emulsion | pH |
|---|---|
| 0 Days | 3.6 |
| 3 Days | 3.5 |
| 7 Days | 3.5 |
| 14 Days | 3.4 |
| 21 Days | 3.7 |
| 28 Days | 3.7 |
| 1 Month | 3.8 |
| 2 Months | 3.8 |

**(11) Phase transition measurement depending on temperature and time**

In order to measure stability, such as phase transition, deterioration and separation, of the Pickering emulsion composition prepared using the composition shown in Table 5, the experiment was performed while maintaining temperatures of 0°C, 25°C, and 45°C for 60 days. The results thereof are shown in Table 14 below. Based on the results of measurement, it was confirmed that L-ascorbic acid became unstable at 45°C and thus turned into a yellow powder. It was confirmed that the white powder was maintained at temperatures of 0°C and 25°C. Therefore, it is concluded that the formulation becomes unstable at high temperatures.

[Table 14]

| Pickering emulsion | 0°C | 25°C | 45°C |
|---|---|---|---|
| 0 Days | ○ | ○ | ○ |
| 3 Days | ○ | ○ | ○ |
| 7 Days | ○ | ○ | × |
| 14 Days | ○ | ○ | × |
| 21 Days | ○ | ○ | × |
| 28 Days | ○ | ○ | × |
| 1 Month | ○ | ○ | × |
| 2 Months | ○ | ○ | × |

The features, structures, effects, and the like exemplified in the aforementioned embodiments may be combined or modified for other embodiments by persons having ordinary knowledge in the art to which the embodiments belong. Therefore, contents related to such combinations and modifications should be interpreted as being included in the scope of the present invention.

**Claims**

1. A cosmetic composition comprising an *Azadirachta indica* leaf extract as an active ingredient and having antibacterial efficacy, anti-inflammatory efficacy and anti-pollution efficacy,
   wherein the anti-pollution efficacy prevents skin adsorption of air pollutants, fine dust or heavy metal particles and protects skin cells.

2. The cosmetic composition of claim 1, wherein the cosmetic composition additionally has antioxidant efficacy, whitening efficacy and wrinkle reduction efficacy.

3. The cosmetic composition of claim 1, wherein the *Azadirachta indica* leaf extract is an *Azadirachta indica* leaf hot-water extract, an *Azadirachta indica* leaf ethanol extract or an *Azadirachta indica* leaf acetone extract.

4. A cosmetic product composition containing an *Azadirachta indica* leaf extract, comprising the cosmetic composition of any one of claims 1 to 3.

5. A Pickering emulsion composition comprising an *Azadirachta indica* leaf extract as an active ingredient, further comprising a powder phase, a water phase and an additive, and having antibacterial efficacy, anti-inflammatory efficacy and anti-pollution efficacy,
   wherein the anti-pollution efficacy prevents skin adsorption of air pollutants, fine dust or heavy metal particles and protects skin cells.

6. The Pickering emulsion composition of claim 5, wherein the Pickering emulsion composition is prepared by subjecting a composition comprising the *Azadirachta indica* leaf extract extracted using hot water, ethanol or acetone, the powder phase, the water phase and the additive to pulverization at 10,000 rpm to 20,000 rpm for 30 sec to 5 min and then mixing at 300 to 800 rpm for 1 to 5 min.

7. The Pickering emulsion composition of claim 5, wherein the Pickering emulsion composition additionally has antioxidant efficacy, whitening efficacy and wrinkle reduction efficacy.

FIG. 1

```
                    ┌─────────────────────────────────┐
                    │           Extraction            │
                    └─────────────────────────────────┘

        ┌───────────────────────────────────────────────────────┐
        │              Azadirachta indica                       │
        └───────────────────────────────────────────────────────┘
            │                      │                      │
            ▼                      ▼                      ▼
  ┌──────────────────┐  ┌──────────────────┐  ┌──────────────────┐
  │   Extraction     │  │   Extraction     │  │   Extraction     │
  │ (70% ethanol room│  │  (Water 99 ℃     │  │ (70% acetone room│
  │  Temperature,    │  │  Temperature,    │  │  Temperature,    │
  │ 24 hr × 3 times) │  │  1hr × 3 times)  │  │ 24 hr × 3 times) │
  └──────────────────┘  └──────────────────┘  └──────────────────┘
            │                      │                      │
            ▼                      ▼                      ▼
  ┌──────────────────┐  ┌──────────────────┐  ┌──────────────────┐
  │   Supernatant    │  │   Supernatant    │  │   Supernatant    │
  └──────────────────┘  └──────────────────┘  └──────────────────┘
            │                      │                      │
            ▼                      ▼                      ▼
  ┌──────────────────┐  ┌──────────────────┐  ┌──────────────────┐
  │    Filtration    │  │    Filtration    │  │    Filtration    │
  └──────────────────┘  └──────────────────┘  └──────────────────┘
            │                      │                      │
            ▼                      ▼                      ▼
  ┌──────────────────┐  ┌──────────────────┐  ┌──────────────────┐
  │   Evaporation    │  │   Evaporation    │  │   Evaporation    │
  └──────────────────┘  └──────────────────┘  └──────────────────┘
            │                      │                      │
            ▼                      ▼                      ▼
  ┌──────────────────┐  ┌──────────────────┐  ┌──────────────────┐
  │  Freeze drying   │  │  Freeze drying   │  │  Freeze drying   │
  └──────────────────┘  └──────────────────┘  └──────────────────┘
            │                      │                      │
            ▼                      ▼                      ▼
  ┌──────────────────┐  ┌──────────────────┐  ┌──────────────────┐
  │   70% ethanol    │  │     Water        │  │   70% acetone    │
  │    extracts      │  │    extracts      │  │    extracts      │
  └──────────────────┘  └──────────────────┘  └──────────────────┘
```

EP 3 741 379 A1

FIG. 2a

FIG. 2b

FIG. 2c

FIG. 2d

FIG. 3a

FIG. 3b

FIG. 4a

FIG. 4b

FIG. 5a

FIG. 5b

FIG. 5c

FIG. 6a

FIG. 6b

FIG. 6c

FIG. 6d

FIG. 7a

FIG. 7b

FIG. 8a

FIG. 8b

FIG. 9a

FIG. 9b

FIG. 9c

FIG. 10

FIG. 11a

FIG. 11b

FIG. 11c

FIG. 12

FIG. 13a

FIG. 13b

FIG. 13c

## INTERNATIONAL SEARCH REPORT

| International application No. |
|---|
| PCT/KR2019/018216 |

**A. CLASSIFICATION OF SUBJECT MATTER**

*A61K 36/58(2006.01)i, A61K 9/107(2006.01)i, A61P 17/10(2006.01)i, A61P 17/18(2006.01)i, A61P 31/04(2006.01)i, A61K 8/9789(2017.01)i, A61K 8/06(2006.01)i, A61Q 19/00(2006.01)i, A61Q 19/02(2006.01)i, A61Q 19/08(2006.01)i*

According to International Patent Classification (IPC) or to both national classification and IPC

**B. FIELDS SEARCHED**

Minimum documentation searched (classification system followed by classification symbols)

A61K 36/58; A61K 35/78; A61K 36/185; A61K 36/42; A61K 8/06; A61K 8/37; A61K 8/92; A61K 8/97; A61Q 19/00; B01D 11/00; B01F 17/00; C08J 3/02; A61K 9/107; A61P 17/10; A61P 17/18; A61P 31/04; A61K 8/9789; A61Q 19/02; A61Q 19/08

Documentation searched other than minimum documentation to the extent that such documents are included in the fields searched
Korean utility models and applications for utility models: IPC as above
Japanese utility models and applications for utility models: IPC as above

Electronic data base consulted during the international search (name of data base and, where practicable, search terms used)
eKOMPASS (KIPO internal) & Keywords: Azadirachta indica leaf, extract, cosmetics, Pickering emulsion

**C. DOCUMENTS CONSIDERED TO BE RELEVANT**

| Category* | Citation of document, with indication, where appropriate, of the relevant passages | Relevant to claim No. |
|---|---|---|
| X | KR 10-2011-0037462 A (THEFACESHOP CO., LTD.) 13 April 2011<br>See paragraphs [0022], [0023]; and experimental example 10. | 1-7 |
| X | KR 10-2011-0085999 A (UNILEVER N.V.) 27 July 2011<br>See claims 1-10; and paragraphs [0005], [0024]. | 1-7 |
| X | ADHIKARI, A. et al. Screening of Nepalese crude drugs traditionally used to treat hyperpigmentation: in vitro tyrosinase inhibition. International journal of cosmetic science. 2008, vol. 30, pages 353-360<br>See abstract; and page 355. | 1-7 |
| A | KR 10-1757245 B1 (KOREA ADVANCED INSTITUTE OF SCIENCE AND TECHNOLOGY) 13 July 2017<br>See the entire document. | 1-7 |
| A | KR 10-2003-0002004 A (OH, Sei-goon) 08 January 2003<br>See the entire document. | 1-7 |
| A | JP 2000-237501 A (NIIMU:KK.) 05 September 2000<br>See the entire document. | 1-7 |
| A | KR 10-2015-0082870 A (COSMECCA KOREA CO., LTD.) 16 July 2015<br>See the entire document. | 1-7 |

☐ Further documents are listed in the continuation of Box C. ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 08 APRIL 2020 (08.04.2020) | **08 APRIL 2020 (08.04.2020)** |

| Name and mailing address of the ISA/KR<br>Korean Intellectual Property Office<br>Government Complex Daejeon Building 4, 189, Cheongsa-ro, Seo-gu, Daejeon, 35208, Republic of Korea<br>Facsimile No. +82-42-481-8578 | Authorized officer<br><br>Telephone No. |
|---|---|

Form PCT/ISA/210 (second sheet) (January 2015)

**INTERNATIONAL SEARCH REPORT**
Information on patent family members

International application No.

**PCT/KR2019/018216**

| Patent document cited in search report | Publication date | Patent family member | Publication date |
|---|---|---|---|
| KR 10-2011-0037462 A | 13/04/2011 | None | |
| KR 10-2011-0085999 A | 27/07/2011 | AU 2009-306492 A1 | 29/04/2010 |
| | | AU 2009-306492 B2 | 30/05/2013 |
| | | BR PI0914462 A2 | 27/10/2015 |
| | | CA 2740213 A1 | 29/04/2010 |
| | | CN 102196813 A | 21/09/2011 |
| | | CN 102196813 B | 25/06/2014 |
| | | CN 103893270 A | 02/07/2014 |
| | | EP 2341917 A2 | 13/07/2011 |
| | | EP 2341917 B1 | 05/12/2012 |
| | | ES 2398493 T3 | 19/03/2013 |
| | | JP 2012-506400 A | 15/03/2012 |
| | | JP 5542143 B2 | 09/07/2014 |
| | | KR 10-1676503 B1 | 15/11/2016 |
| | | MX 2011004149 A | 27/06/2011 |
| | | US 2011-0236509 A1 | 29/09/2011 |
| | | US 8545901 B2 | 01/10/2013 |
| | | WO 2010-046316 A2 | 29/04/2010 |
| | | WO 2010-046316 A3 | 22/07/2010 |
| | | ZA 201102641 B | 25/07/2012 |
| KR 10-1757245 B1 | 13/07/2017 | DE 112016003407 T5 | 12/04/2018 |
| | | KR 10-2017-0013544 A | 07/02/2017 |
| | | US 2018-0141015 A1 | 24/05/2018 |
| | | WO 2017-018637 A1 | 02/02/2017 |
| KR 10-2003-0002004 A | 08/01/2003 | None | |
| JP 2000-237501 A | 05/09/2000 | None | |
| KR 10-2015-0082870 A | 16/07/2015 | KR 10-1547528 B1 | 26/08/2015 |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **SHIN JH ; JANG JR ; KANG MJ ; SHIN JH.** Physiological Activity of Five Kinds of Medicinal Plant Extracts with Various Solvents and Their Composites. *Journal of Life Science,* 2018, vol. 28 (3), 320-330